(19) Europäisches Patentamt
European Patent Office
Office européen des brevets

(11) EP 3 773 443 B1

(12) # FASCICULE DE BREVET EUROPEEN

(45) Date de publication et mention de la délivrance du brevet:
**11.03.2026 Bulletin 2026/11**

(21) Numéro de dépôt: **19742419.5**

(22) Date de dépôt: **12.04.2019**

(51) Classification Internationale des Brevets (IPC):
***A61K 8/368*** (2006.01) ***A61Q 19/00*** (2006.01)
***A61P 17/00*** (2006.01)

(52) Classification Coopérative des Brevets (CPC):
**A61K 8/368; A61P 17/00; A61Q 19/005**

(86) Numéro de dépôt international:
**PCT/FR2019/050870**

(87) Numéro de publication internationale:
**WO 2019/197789 (17.10.2019 Gazette 2019/42)**

(54) **COMPOSITION POUR EMPÊCHER OU RALENTIR L'APPARITION DE SIGNES D'INFLAMMATION**

ZUSAMMENSETZUNG ZUR VORBEUGUNG ODER VERLANGSAMUNG DES AUFTRETENS VON ANZEICHEN EINER ENTZÜNDUNG

COMPOSITION FOR PREVENTING OR SLOWING THE APPEARANCE OF SIGNS OF INFLAMMATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.04.2018 FR 1853265**

(43) Date de publication de la demande:
**17.02.2021 Bulletin 2021/07**

(73) Titulaire: **Société d'Exploitation de Produits pour les**
**Industries Chimiques SEPPIC**
**75321 Paris cedex 07 (FR)**

(72) Inventeurs:
- **KERN, Catherine**
  **75321 PARIS CEDEX 07 (FR)**
- **GARCIA, Christine**
  **81100 CASTRES (FR)**
- **GOMBERT, Christian**
  **75321 PARIS CEDEX 07 (FR)**
- **MSIKA, Philippe**
  **75321 PARIS CEDEX 07 (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**WO-A1-2012/098342** **WO-A2-2006/127525**
**CN-A- 106 074 663** **JP-A- 2011 207 799**
**US-A- 4 363 815**

- **JIANG XIAO-WEN ET AL: "Caffeoylquinic acid derivatives from the roots ofArctium lappaL. (burdock) and their structure-activity relationships (SARs) of free radical scavenging activities", PHYTOCHEMISTRY LETTERS, vol. 15, 8 January 2016 (2016-01-08), pages 159 - 163, XP029459897, ISSN: 1874-3900, DOI: 10.1016/J.PHYTOL.2015.12.008**
- **XIAOWEI ZOU ET AL: "Isolation and characterization of two new phenolic acids from cultured cells of Saussurea involucrata", PHYTOCHEMISTRY LETTERS, vol. 7, 1 February 2014 (2014-02-01), AMSTERDAM, NL, pages 133 - 136, XP055523570, ISSN: 1874-3900, DOI: 10.1016/j.phytol.2013.11.002**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet l'utilisation d'une composition comprenant des dérivés d'acides quiniques poly-substitués, et plus particulièrement d'un extrait de la plante *Arctium lappa* comprenant lesdits dérivés pour préparer des formulations à usage topique destinées à empêcher ou à ralentir l'apparition des signes inesthétiques liés à l'inflammation de la peau et/ou du cuir chevelu, et plus particulièrement des peaux réactives et/ou sensibles.

La peau humaine constituant la première image offerte au regard d'autrui, l'amélioration de son aspect est souvent un sujet de préoccupation pour l'être humain. La peau est le reflet soit d'un état de bien-être, souvent associé à une peau nette ou éclatante, soit, a contrario, à un état de fatigue, souvent associé aux effets inesthétiques d'une peau irritée, comme par exemple la présence de rougeurs sur la peau et plus particulièrement sur certaines parties du visage comme les joues, le cou, le front. Ces rougeurs se développent plus particulièrement suite à de nombreux stress extérieurs (comme par exemple des changements de température) et touchent notamment les peaux sensibles et réactives.

La peau est un organe atypique du corps humain, extrêmement fin au regard de son étendue mais également l'organe le plus lourd d'un individu. Une des caractéristiques de la peau réside dans le fait qu'il s'agit d'un organe d'interface, d'un organe limite, entre le milieu intérieur (corps humain) et le milieu extérieur. De ce fait, et avec la flore qui la recouvre et l'habite, la peau est la première barrière de protection de l'organisme humain.

En raison de sa position d'interface avec le milieu extérieur, la peau est soumise à des sollicitations quotidiennes nombreuses, telles que par exemple le contact avec des vêtements, les changements de températures, les changements de degrés d'hygrométrie, les changements de pression, voire à des agressions, comme par exemple le contact avec certains produits chimiques présentant ou pouvant présenter un caractère très acide, ou très basique, ou irritant, avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui. Par conséquent, l'amélioration de son aspect est un sujet de préoccupation constant pour les êtres humains. La peau est le reflet d'un état de bien-être, souvent associé à la jeunesse, et *a contrario* d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.
A la périphérie de l'épiderme, se trouve une couche cornée supérieure, nommée le *stratum corneum,* qui est la première couche de l'épiderme à subir les stress d'origine externe, comme les variations de conditions climatiques extérieures (température, pression, hygrométrie) ou les sollicitations mécaniques.

Le *stratum corneum* est plus particulièrement en contact avec le microbiote cutané.

Par « microbiote cutané », on désigne au sens de la présente demande une population de micro-organismes, spécialisés ou opportunistes, comme par exemple les bactéries, les champignons, les levures..., qui vit à la surface de la peau.

Le microbiote cutané ne peut pas être défini de façon spécifique et généralisé pour l'ensemble des individus. Depuis le lancement en 2007 du projet « Human Microbiome Project » (HMP) du National Institute of Health, les chercheurs ont pu observer de grandes variations topographiques du microbiote humain ainsi que de grandes différences selon les individus.

Au moins dix-neuf *phyla* ont été identifiés dont les quatre principaux sont Actinobacteria (51,8%), Firmicutes (24,4%), Proteobacteria (16,5%) et Bacteroidetes (6,3%). Les genres majoritairement identifiés sont *Corynebacterium, Propionibacterium* et *Staphylococcus.* L'abondance de chaque groupe est fortement dépendante des différentes localisations. Les organismes fongiques isolés sur la peau sont du genre *Malassezia* spp. Par ailleurs, des acariens du genre Demodex sont également présents et résident dans les unités pilo-sébacées, le plus souvent de la surface du

visage.

Ce microbiote s'alimente à la fois de molécules excrétées par la peau (lipides, protéines...), et de composés sécrétés par les communautés de micro-organismes mettant en évidence une réelle coopération au sein de ce microbiote. De plus, cette relation avec l'hôte constitue une véritable symbiose.

Les bactéries peuvent être commensales lorsqu'elles vivent au contact du revêtement cutanéo-muqueux d'un hôte sans entraîner de dommages. Un équilibre s'installe alors entre l'individu et les flores diverses commensales de la peau et des muqueuses, mais cet équilibre est sans cesse menacé par les agressions physiques ou chimiques subies par le *stratum corneum,* comme par exemple la pollution, les variations de température, les rayonnements ultra-violet, l'utilisation intensive de produits tensioactifs détergents, le stress, etc.... A côté de ces bactéries commensales, se trouvent les bactéries opportunistes, indésirables et/ou pathogènes.

*Staphylococcus epidermidis (S. epidermidis)* constitue plus de 90% de la flore résidente en aérobie présente dans le *stratum corneum.* La flore résidente est aussi composée de bactéries anaérobie appartenant à la division des Actinobactéries comme *Propionibacterium acnes (P. acnes),* fréquemment retrouvés au niveau des zones sébacées, comme par exemple le dos, le visage et le cuir chevelu.

Alors que la flore normale de la peau constitue une défense pour l'hôte, une augmentation ou une réduction de la composition bactérienne (dysbiose) conduit à l'inflammation cutanée et peut être à l'origine du développement et de la manifestation visible de rougeurs sur la peau et/ou le cuir chevelu, plus particulièrement sur les peaux sensibles et réactives.

Les peaux sensibles se définissent par une réactivité particulière de la peau. Cette réactivité cutanée se traduit classiquement par la manifestation de signes d'inconfort, comme les rougeurs, en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultraviolets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations plus ou moins douloureuses ressenties dans une zone cutanée comme les picotements, fourmillements, démangeaisons, brûlures, échauffements, inconforts, tiraillements, etc. On sait aujourd'hui que ces réactions d'irritation et d'intolérance cutanée sont notamment liées à des mécanismes inflammatoires

Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Au sens de la présente invention, les cuirs chevelus "sensibles" ont une sémiologie clinique plus univoque : les sensations de démangeaisons et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Il est connu que la colonisation du follicule pilo-sébacé par *P. acnes* est un facteur important pour la réaction inflammatoire dans l'acné vulgaire. De fait, l'acné n'est pas *sensu stricto* une maladie infectieuse car ce germe exerce d'abord une action inflammatoire, liée à ses très nombreuses sécrétions enzymatiques et chimiques et aux réactions immunologiques qu'il provoque. Ainsi, *P. acnes* stimule la production par les sébocytes, les kératinocytes et les leucocytes (lymphocytes et monocytes) de nombreuses cytokines inflammatoires (IL-1$\alpha$, IL1$\beta$, IL-6, IL-8, IL-10, IL-12, IL-17, IL-18, TNF-$\alpha$, GM-CSF et IFN-$\gamma$) ainsi que des peptides antimicrobiens (défensines et cathélicidines), des métalloprotéinases matricielles, des espèces réactives de l'oxygène et d'autres produits impliqués dans la réaction inflammatoire.

De plus, *P. acnes* sécrète une lipase qui hydrolyse les triglycérides du sébum en acides gras libres qui sont irritants et chimiotactiques pour les neutrophiles.

Parmi les extraits de végétaux que l'on peut utiliser pour leurs actions sur le microbiote humain, on peut citer un extrait lyophilisé de feuilles de bardane (ou *Arctium lappa*) pour lequel une activité antibactérienne a été mise en évidence et plus particulièrement une activité contre des micro-organismes oraux, se montrant plus efficaces contre des bactéries associées à des pathogènes endodontiques tels que *Bacillus subtilis, Candida albicans, Lactobacillus acidophilus* et

*Pseudomonas aeruginosa* (1).

Il est également décrit que les feuilles de bardane sont aussi un remède topique possible pour des problèmes de peau tels que l'eczéma, l'acné et le psoriasis (1).

Il est également décrit dans la littérature que les extraits de feuilles de bardane montrent des activités anti-microbiennes (2).

La demande de brevet internationale publiée sous le numéro WO 2006/127525 A2 décrit une composition, comprenant des composés de cinnamate isolés du thé maté, comprenant des esters de caféoyle tels que l'acide 5-caféoylquinique, l'acide 3,5-dicafféoylquinique, l'acide 3, 4, dicafféoylquinique, destinée à inhiber l'activité du protéasome.

La demande de brevet chinois publiée sous le numéro CN106074663 A décrit une composition d'extraits de plantes comprenant un extrait de bois de Milo, un extrait de racines de bardane, et un extrait de chèvrefeuille, et décrit plus particulièrement que l'extrait de racines de Bardane traite les peaux sèches, le prurit aigu, l'inflammation, les cicatrices et d'autres symptômes, en inhibant des facteurs inflammatoires induits par différentes causes (stress externe ou facteurs génétiques), améliorant l'activité immunitaire et l'activité anti-oxydante de la peau, apaisant et réparant la peau.

La demande de brevet américain publiée sous le numéro US20170136077 divulgue qu'un certain nombre d'extraits de végétaux, parmi lesquels un extrait racinaire de bardane, un extrait racinaire d'*Epilobium angustifolium,* un extrait de *Cystoseira amentacea* favorisent la réduction de la production des cytokines IL-8, IL-1 et TNF-$\alpha$ par des kératinocytes stimulés par du Phorbol 12-myristate 13-acétate (ou « PMA »).

Ce modèle est connu pour évaluer l'effet anti-inflammatoire d'ingrédients car le PMA est un activateur de la voie protéine kinase C qui a un effet inflammatoire général sur les cellules.

Dans le cadre de leurs recherches concernant de nouveaux actifs cosmétiques pour la prévention et/ou le traitement des signes des effets inesthétiques liés à l'inflammation cutanée, comme par exemple les rougeurs sur la peau et/ou le cuir chevelu, plus particulièrement sur les peaux sensibles et réactives, les inventeurs se sont attachés à développer une nouvelle solution technique fondée sur l'utilisation d'une composition comprenant des acides quiniques poly-substitués (ou « QPS »), sur l'utilisation d'extraits de racines de Bardane comprenant lesdites QPS, obtenus par un procédé comprenant une étape préalable de culture en aéroponie de ladite Bardane, pour présenter des effets d'hydratation de la peau humaine.

Selon un premier aspect, l'invention a pour objet l'utilisation d'une composition ($C_1$) pour empêcher ou ralentir l'apparition des signes inesthétiques liés à l'inflammation de la peau et/ou du cuir chevelu, ou bien de les éliminer, avec la composition (C1) comprenant pour 100% de sa masse :

**a)** - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;

**b)** - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

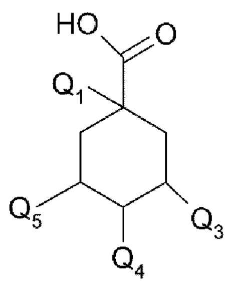

(I),

dans laquelle $Q_1$, $Q_2$, $Q_3$, $Q_4$ et $Q_5$ représentent indépendamment les uns des autres, le radical hydroxyle ou un ses sels ou un radical choisi parmi :

(i) - Le radical caféoyle de formule (II) :

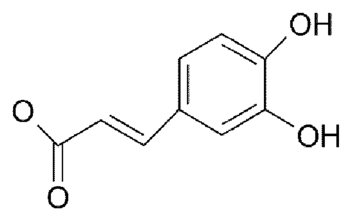

(II)

**(ii)** - Le radical maloyle de formule (IIIa) ou (IIIb) :

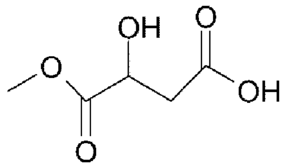

(IIIa)

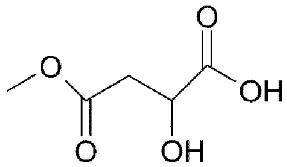

(IIIb) ;

**(iii)** - Le radical caféoyl maloyle de formule (IVa) ou (IVb) :

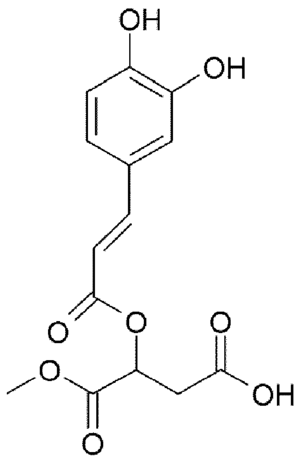

(IVa)

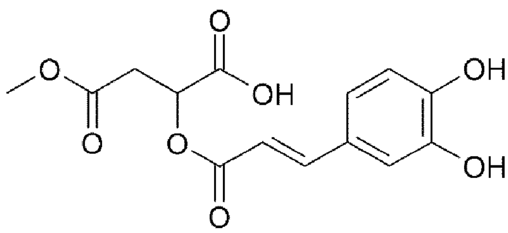

(IVb)

**(iv)** - Le radical maloyl caféoyle de formule (Va), (Vb), (Vc) ou (Vd),

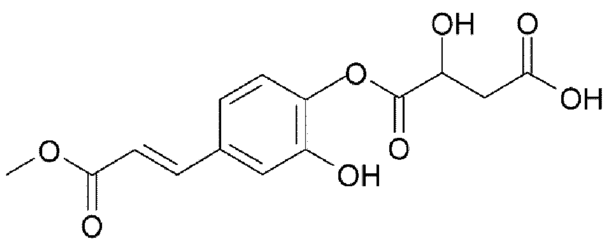

(Va)

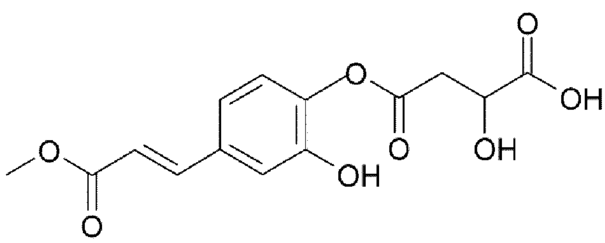

(Vb)

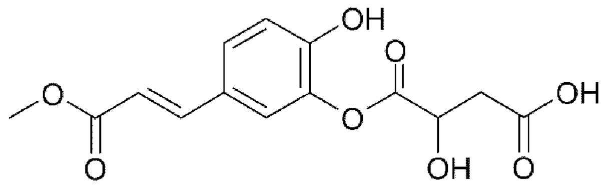

(Vc),

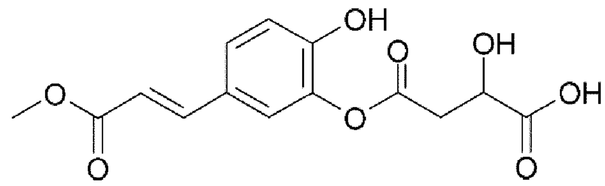

(Vd)

étant entendu qu'au moins un de ces radicaux $Q_1$, $Q_2$, $Q_3$, $Q_4$ et $Q_5$ ne représente ni le radical -OH ; ni un de ses sels ;
ladite composition (ES) comprenant au moins :

- Au moins un composé de formule (Ia) correspondant à la formule (I) pour laquelle Q1 représente le radical maloyle de formule (IIIa) ou de formule (IIIb) et Q3 et Q4 et Q5 identiques, représentent chacun le radical caféoyle de formule (II) ;
- Un composé de formule (Ib) correspondant à la formule (I) pour laquelle Q1 représente le radical caféoylmaloyle de formule (IVa) ou de formule (IVb), Q3 et Q5 représentent chacun le radical caféoyle de formule (II) et Q4 représente le radical hydroxyle, et
- Au moins un composé de formule (Ic) choisi parmi :
    - Le composé de formule (Ic1) correspondant à la formule (I) pour laquelle Q1 et Q5 représentent chacun le radical caféoyle de formule (II), Q3 représente le radical hydroxyle et Q4 représente le radical caféoylmaloyle de formule (IVa) ou de formule (IVb) ; et
    - Le composé de formule (Ic2) correspondant à la formule (I) pour laquelle Q1 et Q4 représentent le radical caféoyle de formule (II), Q3 représente le radical hydroxyle et Q5 représente le radical caféoylmaloyle de formule (IVa) ou de formule (IVb), et
    - **c)** - De 20,0% massique à 35,0% massique d'eau.

Au sens de la présente invention, par « signes inesthétiques liés à l'inflammation de la peau et/ou le cuir chevelu », on entend au sens de l'invention, toutes modifications de l'aspect extérieur de la peau ou du cuir chevelu dues à une inflammation de ladite peau ou ledit cuir chevelu, comme par exemple les inflammations se manifestant par des rougeurs cutanées et/ou du cuir chevelu.

Au sens de la présente invention, l'expression «ladite quantité massique $x_1$ étant exprimée en équivalent massique de l'acide 1-O-(2-caféoyl)maloyl-3,5-O-dicaféoyl quinique » signifie que la quantité massique $x_1$ a été déterminée par la mise en œuvre d'une méthode analytique quantitative de type UHPLC-MS (« Ultra High Performance Liquid Chromatography-Mass Spectra), utilisant comme étalon de référence un standard de l'acide 1-O-(2-caféoyl)maloyl-3,5-O-dicaféoyl quinique préalablement isolé et purifié à une teneur supérieure ou égale à 99%.

Une telle analyse quantitative de type UHPLC-MS a été réalisée avec un appareillage de type UHPLC-MS Shimadzu_Nexera_LCMS 2020, équipé d'un détecteur à barrette de diode et réglé à une longueur d'onde de 330 nanomètres, d'une colonne de type Kinetex 2,6u XB-C18 100A. 100 x 2,1, et mettant en œuvre une phase mobile A composée d'eau et de 0,1% massique d'acide formique et une phase mobile B constituée par l'acétonitrile,

Selon un aspect plus particulier de la présente invention, dans la composition (ES) telle que définie précédemment, le composé de formule (Ia) correspondant à la formule (I) telle que définie précédemment et pour laquelle $Q_1$ représente le radical maloyl de formule (IIIa), $Q_3$ et $Q_4$ et $Q_5$, identiques, représentent le radical caféoyl de formule (II).

Selon un aspect plus particulier de la présente invention, dans la composition (ES) telle que définie précédemment, le composé de formule (Ia) correspondant à la formule (I) telle que définie précédemment et pour laquelle $Q_1$ représente le radical maloyl de formule (IIIb), $Q_3$ et $Q_4$ et $Q_5$, identiques, représentent le radical caféoyl de formule (II).

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVa) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVb) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$, identiques, représentent le radical caféoyl de formule (II) ; $Q_3$ représente le radical -OH ; $Q_4$ représente le radical caféoylmaloyl de formule (IVa).

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$, identiques, représentent le radical caféoyl de formule (II) ; $Q_3$ représente le radical -OH ; $Q_4$ représente le radical caféoylmaloyl de formule (IVb).

Selon un aspect plus particulier de la présente invention, dans la composition (ES) telle que définie précédemment, le composé de formule (Ic) est le composé de formule ($Ic_2$), correspondant à la formule (I) telle que définie précédemment et pour laquelle $Q_1$ et $Q_4$, identiques, représentent le radical caféoyl de formule (II) ; $Q_3$ représente le radical -OH ; $Q_5$ représente le radical caféoylmaloyl de formule (IVa).

Selon un aspect plus particulier de la présente invention, dans la composition (ES) telle que définie précédemment, le composé de formule (Ic) est le composé de formule ($Ic_2$), correspondant à la formule (I) telle que définie précédemment et pour laquelle $Q_1$ et $Q_4$, identiques, représentent le radical caféoyl de formule (II) ; $Q_3$ représente le radical -OH ; $Q_5$ représente le radical caféoylmaloyl de formule (IVb).

Selon un aspect plus particulier de la présente invention, la composition (ES) telle que définie précédemment comprend au moins :

- un composé de formule (Ia) telle que définie précédemment pour laquelle $Q_1$ représente le radical maloyl de formule (IIIa) ou le radical maloyl de formule (IIIb), et
- un composé de formule (Ib) telle que définie précédemment et pour laquelle $Q_1$ représente le radical caféoylmaloyl de formule (IVa) ou le radical caféoylmaloyl de formule (IVb), et
- un composé de formule ($Ic_1$) telle que définie précédemment et pour laquelle $Q_4$ représente le radical caféoylmaloyl de formule (IVa) ou le radical caféoylmaloyl de formule (IVb).

Selon un aspect plus particulier de la présente invention, la composition (ES) telle que définie précédemment comprend au moins :

- un composé de formule (Ia) telle que définie précédemment pour laquelle $Q_1$ représente le radical maloyl de formule (IIIa) ou le radical maloyl de formule (IIIb), et
- un composé de formule (Ib) telle que définie précédemment et pour laquelle $Q_1$ représente le radical caféoylmaloyl de formule (IVa) ou le radical caféoylmaloyl de formule (IVb), et
- un composé de formule ($Ic_2$) telle que définie précédemment et pour laquelle $Q_5$ représente le radical caféoylmaloyl de formule (IVa) ou de formule (IVb).

Selon un aspect particulier de la présente invention, le solvant organique ($SO_1$) présent dans la composition (C1) telle que définie précédemment est choisi parmi les éléments du groupe constitué par le 1,2-propanediol, le 1,3-propanediol, et le 2-méthyl-2,4-pentanediol.

Selon un autre aspect particulier de la présente invention, la composition ($C_1$) comprend pour 100% de sa masse :

- De 60,0% massique à 75,0% massique de 1,2-propanediol,
- De 0,1% massique à 2,0% massique d'une composition (ES) comprenant une quantité massique $x_1$, exprimée en équivalent massique de l'acide 1-O-(2-caféoyl)maloyl-3,5-O-dicaféoyl quinique, supérieure ou égale à 200 mg/ d'au moins le composé de formule (Ia) tel que défini à la revendication 2, et d'au moins le composé de formule (Ib) tel que défini à la revendication 2, et d'au moins le composé de formule (Ic) tel que défini à la revendication 2
- De 20,0% massique à 35,0% massique d'eau.

La composition (C1) utilisée dans le cadre de la présente invention peut être préparée par simple mélange de ses constituants, à température comprise entre 20°C et 60°C, plus particulièrement entre 20°C et 40°C, et encore plus particulièrement entre 20°C et 30°C , et sous agitation mécanique de type ancre à une vitesse comprise entre 50 tours/minute et 150 tours/minute.

Plus précisément la composirion (C1) utilisée dans le cadre de l'invention peut être préparée à partir d'un procdé

comprenant les étapes successives suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/mélange ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;

- Une étape c) de séparation des racines de la biomasse à l'issue du traitement défini à l'étape b), pour isoler une phase liquide ($L_1$) ;
- Une étape d) d'immersion de ladite biomasse ($BM_2$) issue de l'étape c) dans ledit milieu ($S_1$); dans un ratio biomasse ($BM_2$)/mélange ($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$),
- Une étape f) de filtration de ladite phase liquide ($L_3$) obtenue à l'étape d), pour isoler une phase liquide liquide ($L_3$),
- Une étape g) de mélange des dites phases liquides ($L_1$) et ($L_3$), puis si nécessaire d'addition d'eau et/ou dudit solvant organique ($SO_1$), de façon à obtenir la composition ($C_1$) attendue.

L'étape a) de mise en culture en conditions hors sol (ou dite en aéroponie) de la plante *Arctium lappa* se réalise selon les conditions standard connues de l'homme du métier, et plus particulièrement celles concernant l'influence de la teneur en azote (2) (3) (4) (5) (6), et de la teneur en phosphore et en potassium présentes dans le milieu de culture. L'étape a) de mise en culture en conditions hors sol se réalise donc en optimisant le ratio azote/phosphore/potassium présent dans le milieu nutritif, et en optimisant le paramètre d'électroconductivité d'un tel milieu nutritif.

L'étape a) est généralement conduite à une température comprise entre 20°C et 40°C, pendant une durée comprise entre 4 et 10 semaines, de façon à obtenir une biomasse ($BM_1$), en quantité importante notamment au niveau racinaire ; l'étape a) est arrêtée lorsque la croissance de la biomasse ($BM_1$) n'est plus observée.

La présente invention a aussi pour objet un procédé pour empêcher ou pour ralentir l'apparition des signes inesthétiques liés à l'inflammation de la peau et/ou du cuir chevelu, ou bien de les éliminer caractérisé en ce qu'il comprend au moins une étape $a_1)_2$) comprenant au moins un excipient cosmétiquement acceptable (E) et une composition ($C_1$) consistant en, pour 100% de sa masse :

a) de 60,0% massique à 75,0% massique de 1,2-propanediol,
b) de 0,1% massique à 2,0% massique d'une composition (ES) comprenant une quantité massique $x_1$, exprimée en équivalent massique de l'acide 1-O-(2-caféoyl)maloyl-3,5-O-dicaféoyl quinique, supérieure ou égale à 200 mg/ d'au moins le composé de formule (Ia) tel que défini à la revendication 2, et d'au moins le composé de formule (Ib) tel que défini à la revendication 2, et d'au moins le composé de formule (Ic) tel que défini à la revendication 2 c) de 20,0% massique à 35,0% massique d'eau.

Par « quantité efficace », on désigne, dans la définition du procédé tel que défini ci-dessus, une quantité telle qu'elle permette la diminution de l'intensité, ou le ralentissement de l'apparition, ou l'élimination, des signes inesthétiques liés à l'inflammation de la peau et/ou du cuir chevelu, et plus particulièrement des rougeurs. Généralement on utilisera une quantité d'environ 1 gramme à 5 grammes d'une composition à usage topique ($C_2$) telle que définie précédemment.

L'expression "à usage topique" utilisée dans la définition de la composition ($C_2$) objet de la présente invention, signifie que ladite composition ($C_2$) est mise en œuvre par application sur la peau, qu'il s'agisse d'une application directe ou d'une application indirecte lorsque ladite composition ($C_2$) selon l'invention est imprégnée sur un support destiné à être mis en contact avec la peau (papier, lingette, textile, dispositif transdermique, etc...).

Ladite composition ($C_2$) est généralement étalée sur la surface de la peau à traiter, puis la peau est massée quelques instants.

L'expression « cosmétiquement acceptable » utilisée dans la définition de la composition ($C_2$) objet de la présente invention, signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les

maintenir en bon état.

La composition ($C_2$) à usage topique objet de la présente invention, se présente généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition ($C_2$) à usage topique objet de la présente invention peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, la composition ($C_2$) à usage topique objet de la présente invention comporte également des excipients et ou des principes actifs habituellement mis en œuvre dans le domaine des formulations à usage topique, en particulier cosmétiques, dermocosmétiques, pharmaceutiques ou dermopharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacificants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

Comme exemples de tensioactifs moussants et/ou détergents que l'on peut associer à la composition ($C_1$), on peut citer les tensioactifs moussants et/ou détergents anioniques, cationiques, amphotères ou non ioniques.

Parmi les tensioactifs anioniques moussants et/ou détergents que l'on peut associer à la composition ($C_1$), on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alphaoléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents que l'on peut associer à la composition ($C_1$), on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut associer à la composition ($C_1$), on peut citer particulièrement les dérivés d'ammoniums quaternaires.

Parmi les tensioactifs non ioniques moussants et/ou détergents que l'on peut associer à la composition ($C_1$), on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à la composition ($C_1$), on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer à la composition ($C_1$), on peut citer les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

$$CH_2=C(R'_3)-C(=O)-[CH_2-CH_2-O]n'-R'_4 \qquad (VIII)$$

dans laquelle $R'_3$ représente un atome d'hydrogène ou un radical méthyle, R'4 représente un radical alkyle linéaire ou ramifié comportant de huit à trente atomes de carbone et n' représente un nombre supérieur ou égal à un et inférieur ou égal à cinquante.

Comme exemples d'agents épaississants et/ou gélifiants que l'on peut associer à la composition ($C_1$), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à la composition ($C_1$), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à la composition ($C_1$), on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à la composition ($C_1$), on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à la composition ($C_1$), on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à la composition ($C_1$), on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à la composition ($C_1$), on peut citer les xylènes sulfonates, les cumènes sulfonates, l'hexylpolyglucoside, le 2-éthylhexylpolyglucoside, le n-heptylpolyglucoside.

Comme exemples d'agents tensioactifs émulsionnants que l'on peut associer à la composition ($C_1$), on peut citer les tensioactifs non ioniques, des tensioactifs anioniques, des tensioactifs cationiques.

Comme exemples de tensioactifs non-ioniques émulsionnants que l'on peut associer à la composition ($C_1$), on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkylpolyglycosides tels que décrits précédemment, par exemple les compositions commercialisées sous les noms MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples de tensioactifs anioniques que l'on peut associer à la composition ($C_1$), on peut citer le glycéryl stéarate citrate, le cétéarylsulfate, les savons comme le stéarate de sodium ou le stéarate de triéthanolammonium, les dérivés N-acylés d'acides aminés salifiés, par exemple le stéaroyl glutamate.

Comme exemples de tensioactifs cationiques émulsionnants que l'on peut associer à la composition ($C_1$), on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer à la composition ($C_1$), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer à la composition ($C_1$), on peut citer des dérivés N-acylés d'acides aminés, comme la lauroyl lysine commercialisée sous l'appellation AMINOHOPE™LL, l'octenyl starch

succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica

Comme exemples d'agents déodorants que l'on peut associer à la composition ($C_1$), on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à la composition ($C_1$), on peut citer les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à la composition ($C_1$), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à la composition ($C_1$), on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le $\omega$-undecelynoyl phénylalanine commercialisé sous l'appellation SEPIWHITE™MSH, le SEPICALM™VG, le mono ester et/ou le diester de glycérol du $\omega$-undecelynoyl phénylalanine, les $\omega$-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits

végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI : Butylene glycol, Palmitoyl Tripeptide-40) par la société LIPOTEC, les sucres et les dérivés de sucres par exemple le produit commercialisé sous le nom de marque Tanositol™ (nom INCI : inositol) par la société Provital, le produit commercialisé sous le nom de marque Thalitan™ (ou Phycosaccharide™ AG) par la société CODIF international (nom INCI : Aqua and Hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoïdes de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP1515688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux

cités dans la demande de brevet européen publiée sous le numéro EP 1 515 688 A2.

Comme exemples d'agents antioxydants que l'on peut associer à la composition ($C_1$), on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE□ GL 47S commercialisé par la société Akzo Nobel sous le nom INCI : Tetrasodium Glutamate Diacetate.

Comme exemples de filtres solaires que l'on peut associer à la composition ($C_1$), on peut citer tous ceux figurant dans la directive cosmétique 76/768/CEE modifiée annexe VII.

Parmi les filtres organiques solaires que l'on peut associer à la composition ($C_2$) à usage topique objet de la présente invention telle que définie précédemment, on peut citer la famille des dérivés de l'acide benzoïque comme les acides para-aminobenzoïques (PABA), notamment les esters de monoglycérol de PABA, les esters éthyliques de N,N25 propoxy PABA, les esters éthyliques de N,N-diéthoxy PABA, les esters éthyliques de N,Ndiméthyl PABA, les esters méthyliques de N,N-diméthyl PABA, les esters butyliques de N,Ndiméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl- 2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de pméthoxy 2-éthylhexyle), le cinnamate de p-méthoxy 2-éthoxyéthyle, le p-cinnamate de méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-5 carboxylate, la 2-hydroxy-4-n-octyloxybenzophénone, la 4-hydroxy-3-carboxybenzophénone ; le 3-(4'-méthylbenzylidène)-d,l-camphre, le 3 (benzylidène)-d,lcamphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl)amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-tbutylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", que l'on peut associer à la composition ($C_2$) à usage topique objet de la présente invention telle que définie précédemment, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a aussi pour objet une composition ($C_1$) telle que définie précédemment, pour son utilisation dans une méthode de traitement thérapeutique visant à diminuer et/ou éliminer les picotements et/ou les fourmillements et/ou les démangeaisons et/ou les échauffements et/ou les rougeurs et/ou l'inconfort cutané et/ou les tiraillements de la peau provoqués par l'inflammation de la peau humaine et/ou le cuir chevelu.

De préférence les picotements et/ou les fourmillements et/ou les démangeaisons et/ou les échauffements et/ou les rougeurs et/ou l'inconfort cutané et/ou les tiraillements accompagnent les pathologies cutanées comme l'urticaire, les dermatites eczémateuses, la rosacée, le psoriasis, l'herpès, les photodermatoses, la dermatite atopique, la dermatite de contact, le lichen, les prurigos, les maladies prurigineuses, les fibroses, les troubles de la maturation du collagène, la sclérodermie, l'eczéma.

Bibliographie :

**(1) :** Chan et al., « A review of the pharmacological effects of Arctium lappa », Inflammopharmacol, 2011, 19:245-254).

**(2) :** Pirvu et al.,"Comparative studies on analytical, antioxidant, and antimicrobial activities of a series of vegetal extracts prepared from eight plant species growing in Romania", J planar Chromato 2014.

Les exemples suivants illustrent l'invention, sans toutefois la limiter.

## A) Exemple de préparation

### $A_1$) Exemple de préparation d'une composition ($C_{1A}$) selon l'invention.

Les plantes de Bardane ou *Arctium lappa* ont préalablement été obtenues par germination de graines pendant une durée de 60 jours dans des conditions standard, de façon à atteindre une taille d'environ 10 à 15 centimètres, puis elles sont dépotées pour être placées dans des conditions de culture « hors-sol » ou en milieu aéroponie.

Les racines des plantes de Bardane sont ainsi trempées dans une solution nutritive se caractérisant par une électro-conductivité comprise entre 1,0 et 1,2 millisiemens, et par un ratio massique N/P/K (Azote/Phosphore/Potassium) apportés par l'engrais d'environ 15/10/30. Cette phase de culture en aéroponie est conduite pendant six semaines à une température régulée à 20°C, et permet d'obtenir un rendement racinaire de 754 grammes par mètre carré.

Les racines fraîches de la biomasse ainsi obtenue sont prélevées et immergées pendant 15 minutes dans un bain comprenant un mélange comprenant pour 100% de sa masse, 70% massique de 1,2-propanediol et 30% massique d'eau distillée, à une température de 25°C ; la valeur du pH de l'eau distillée ayant été préalablement réglé à 2,0 ±0,2 par ajout d'une solution à 75% massique d'acide phosphorique. Le ratio de biomasse racinaire ainsi immergées pour un volume de mélange 1,2-propanediol et d'eau préalablement décrit, s'élève à 1,0 kg de biomasse racinaire pour 1 litre de de mélange 1,2-propanediol et d'eau.

A l'issue de l'immersion, les plantes sont sorties de leur bain d'exsudation ($L_1$) qui est conservé et les racines sont égouttées, puis coupées, puis la biomasse restante est remise à macérer pendant une durée de 48 heures dans un bain comprenant un mélange comprenant pour 100% de sa masse, 70% massique de 1,2-propanediol et 30% massique d'eau distillée, à une température de 25°C ; la valeur du pH de l'eau distillée ayant été préalablement réglé à 2,0 ±0,2 par ajout d'une solution à 75% massique d'acide phosphorique. Le ratio de biomasse racinaire ainsi immergées pour un volume de mélange 1,2-propanediol et d'eau préalablement décrit, s'élève à 0,5 kg de biomasse pour 1 litre de de mélange 1,2-propanediol et d'eau.

A l'issue de cette phase de macération, la biomasse est séparée du liquide de macération ($L_2$), ledit liquide ($L_2$) étant par la suite filtré avec un filtre poche de 50 micromètres.

Les liquides ($L_1$) et ($L_2$) sont par la suite regroupés, et une quantité nécessaire de 1,2-propanediol est rajoutée pour ajuster sa teneur massique à 70%, pour obtenir le liquide ($L_3$), qui est par la suite filtré sous membrane de 1 micromètre de façon à le clarifier, et enfin sous filtration stérilisante avec une membrane à 0,2 micromètre, de façon à atteindre la composition ($C_{1A}$).

### $A_2$) Exemple de préparation d'une composition comparative ($C_{comp}$).

Les plantules issues du même lot de graines que celle utilisées pour obtenir les plantules cultivées par la suite en aéroponie (exemple $A_1$), sont utilisées pour une culture en terre desdites plantules pendant une durée de six semaines.

A l'issue de cette période, les plants sont dépotées, les racines fraîches nettoyées, coupées et broyées, puis ledit broyat obtenu est extrait selon un procédé d'extraction liquide-solide conventionnel (macération, agitation, filtration) à l'aide d'un mélange solvant 1,2-proapanediol/eau distillée de 70/30, à une température de 25°C, avec un ratio massique racines fraiches/milieu solvant de 0,5 kg de biomasse pour 1 litre de de mélange 1,2-propanediol et d'eau ; la valeur du pH de l'eau distillée ayant été préalablement réglé à 2,0 ±0,2 par ajout d'une solution à 75% massique d'acide phosphorique.

A l'issue de cette phase d'extraction, la biomasse est séparée du liquide qui est ensuite filtré avec un filtre poche de 50 micromètres, puis avec une membrane de 1 micromètre de façon à le clarifier, et enfin sous filtration stérilisante avec une membrane à 0,2 micromètre, de façon à atteindre la composition ($C_{Comp}$).

### $A_3$) Caractérisation analytique de la composition ($C_{1A}$) selon l'invention et de la composition comparative ($C_{comp}$).

La composition ($C_{1A}$) selon l'invention et de la composition comparative ($C_{comp}$) ont été caractérisées analytiquement et les caractéristiques sont comprises dans le Tableau 7 ci-dessous.

**Tableau 7**

| Caractéristiques analytiques | Méthode analytique | Composition ($C_{1A}$) selon l'invention | Composition comparative ($C_{comp}$) |
|---|---|---|---|
| Apparence | Visuelle | Liquide jaune | Liquide jaune |

(suite)

| Caractéristiques analytiques | Méthode analytique | Composition ($C_{1A}$) selon l'invention | Composition comparative ($C_{comp}$) |
|---|---|---|---|
| Teneur en 1,2 propanediol | Chromatographie phase gaz (espace de tête) | 71,4% | 70,0% |
| pH | NFT 73-206 | 3,1 | 3,2 |
| Extrait sec dû à la plante en % massique | Etuve 105°C, 12 heures | 0,21% | 1,12% |
| Eau en % massique | (Norme NFT 73201) | 28,39% | 28,88% |
| Teneur totale en composés de formule (Ia), de formule (Ib), de formule ($Ic_1$) et de formule ($Ic_2$) exprimée en milligramme/gramme d'extrait sec dû à la plante | UHPLC-MS<br>Appareil : Shimadzu Nexera X2<br>Colonne : Waters Xterra RP C18 ; 250x4,6 mm<br>Phase mobile (avec gradient):<br>A) Eau + acide formique<br>B) Acétonitrile<br>Détecteur UV (330 nm) | 613,3 mg/g | 169,5 mg/g |

**B) Mise en évidence des propriétés actives des compositions ($C_{1A}$) selon l'invention et ($C_{comp}$) comparative.**

**$B_1$) Mise en évidence de la prévention de l'altération de la fonction barrière de la peau, sur des épidermes reconstruits humains.**

*B1.1. Principe de la méthode*

Les souches *Staphylococcus epidermidis* et *Staphylococcus aureus* ont été cultivées en milieux BHI (Brain Heart Infusion) et NB (Nutrient Broth) respectivement, à 37°C, pendant 24 heures. Des épidermes reconstruits humains de 0,5 $cm^2$ de surface, cultivés à 37°C et sous 5% $CO_2$, ont été colonisés tout d'abord avec la souche *Staphylococcus epidermidis* pendant une durée de 6 heures, puis ont été colonisés avec la souche *Staphylococcus aureus* pendant 24 heures.

La composition ($C_{1A}$) selon l'invention (1% v/v) a été ajoutée sur les épidermes reconstruits humains en même temps que la souche *Staphylococcus epidermidis* puis à nouveau avec la souche *Staphylococcus aureus*.
La fonction barrière des épidermes reconstruits humains ainsi traités a été évaluée :

- par une mesure de la résistance électrique trans-épithéliale, ou TEER (Trans Epithelial Electrical Resistance), des épidermes reconstruits humains et
- par une évaluation histologique desdits épidermes reconstruits humains, et plus particulièrement par coloration hématoxyline et éosine, et par un « score » de ladite coloration.

Dans le cadre de l'évaluation histologique, les effets sur la fonction barrière des épidermes reconstruits humains ont été évalués par un score histologique sur coloration hématoxyline et éosine qui a été attribué comme suit :

- 0 = standard : aucune modification significative de la morphologie de référence
- 1 = léger : modification significative du *stratum corneum*
- 2 = modéré : modifications significatives du *stratum corneum* et de la couche granuleuse, diminution de la kératohyaline et quelques cellules nécrotiques
- 3 = fort : modifications significatives de la couche basale avec des cellules nécrotiques et des trous intercellulaires et

des oedèmes

- 4 = sévère : perte de la connexion intercellulaire, détachement du tissu du filtre polycarbonate, cellules nécrotiques, absence de marquage spécifique.

Un produit est jugé comme protecteur de la fonction barrière de l'épiderme humain si le score histologique est noté « standard » (score 0) ou « léger » (Score 1).
L'équilibre du microbiote de l'épiderme humain reconstruit, sans ou avec application de la composition ($C_{1A}$), a été évalué par l'étude de la formation des ultrastructures type colonies, biofilms par SEM.

*B.1.2. Résultats*

*B.1.2.1 Résultats obtenus sur la protection de la fonction barrière des épidermes humains reconstruits par mesure de la résistance électrique trans-épithéliale TEER (Trans Epithelial Electrical Resistance).*

Les mesures de la TEER réalisées sur les épidermes humains reconstruits, en fonction des traitements associés sont consignées dans le Tableau 8. Une diminution de la résistance électrique trans-épithéliale (TEER) témoigne d'une dégradation de la fonction barrière de l'épiderme et par conséquent constitue un des facteurs de déshydratation de la peau et des effets inesthétiques que peut provoquer cette déshydratation. On calcule également la **différence de la mesure de la résistance électrique trans-épithéliale de la surface de l'épiderme humain reconstruit après colonisation et avant colonisation (Δ1)**
On calcule également le pourcentage de protection selon la formule :

% Protection = **Δ1 [** Epiderme humain reconstruit colonisé avec *Staphylococcus epidermis* + *Staphylococcus aureus* et traité par la Composition ($C_{1A}$) 1% (v/v)] - **Δ1** [Epiderme humain reconstruit colonisé avec *Staphylococcus epidermidis* + *Staphylococcus aureus* sans ajout de la Composition ($C_{1A}$) ] ) / (**Δ1** [ Epiderme humain reconstruit non traité (Contrôle) ] - **Δ1** [Epiderme humain reconstruit colonisé avec *Staphylococcus epidermidis* + *Staphylococcus aureus* sans ajout de la Composition ($C_{1A}$) ] )

L'analyse statistique des résultats a été menée à l'aide d'un test de Student bilatéral et un seuil de significativité fixé à 5%, en comparant deux à deux les colonisations et traitements effectués.
On considérera qu'une différence entre l'efficacité de deux produits est :

- Significative si $p < 0,05$ ;
- Dite « à la limite de significativité » si $0,05 \leq p < 0,1$ ;
- Et non significative si $p > 0,1$.

**Tableau 8**

| | **Mesure de la résistance électrique trans-épithéliale de la surface de l'épiderme humain reconstruit Avant colonisation avec les bactéries et/ou traitement avec la composition ($C_{1A}$) (en Ohm.cm$^2$)** | **Mesure de la résistance électrique trans-épithéliale de la surface de l'épiderme humain reconstruit Après colonisation avec les bactéries et/ou traitement avec la composition ($C_{1A}$) (en Ohm.cm$^2$)** | **Différence de la mesure de la résistance électrique trans-épithéliale de la surface de l'épiderme humain reconstruit après colonisation/traite ment et avant colonisation/traite ment (Δ1) (en Ohm.cm$^2$)** |
|---|---|---|---|
| Epiderme humain reconstruit non traité (Contrôle) | 8073,33 +/- 1913,60 | 6900,00 +/- 1489,80 | - 1173,33 |

(suite)

| | | | |
|---|---|---|---|
| Epiderme humain reconstruit colonisé avec *Staphylococcus* epidermidis + *Staphylococcus* aureus sans ajout de la Composition ($C_{1A}$) | 8094,44 +/- 1486,73 | 4726,11 +/- 2067,16 | - 3368,33<br>(% versus contrôle = +187% ; p=0,009) |
| Epiderme humain reconstruit colonisé avec *Staphylococcus epidermidis* + *Staphylococcus aureus* et traité par la Composition ($C_{1A}$) 1% (v/v) (0,0136% d'extrait sec de la composition ($C_{1A}$)) | 7647,22 +/- 1566,61 | 5645,56 +/- 911,52 | - 2001,66 (% versus contrôle = +71% ; non significatif)<br>Pourcentage protection = 62% |

Lorsque les épidermes humains reconstruits sont colonisés avec *Staphylococcus epidermidis* et *Staphylococcus aureus,* la différence de TEER mesurée avant et après le début de ladite colonisation est de -3373,33 Ohm.cm$^2$, et présente une augmentation significative de 187 % par rapport aux épidermes humains reconstruits non colonisés et non traités (-1173,33 Ohm.cm$^2$).

Lorsque les épidermes humains reconstruits sont mis en contact avec la Composition ($C_{1A}$) en même temps que leur colonisation avec *Staphylococcus epidermidis* et *Staphylococcus aureus,* la différence de TEER mesurée avant et après le début de ladite colonisation est de -2001,66 Ohm.cm$^2$, ce qui représente une augmentation non significative de 71 % par rapport aux épidermes humains reconstruits non colonisés et non traités (-1173,33 Ohm.cm$^2$) et une protection de 62% par rapport aux épidermes humains reconstruits colonisés avec *Staphylococcus epidermidis* et *Staphylococcus aureus.*

Il en résulte que l'application sur la peau d'une composition comprenant la Composition ($C_{1A}$) permet de prévenir la dégradation de la fonction barrière de l'épiderme de la peau, avant que celle-ci soit soumise à l'action de bactéries connues pour leurs effets de dégradation de la fonction barrière dudit épiderme de la peau.

*B.1.2.2 Résultats obtenus sur la protection de la fonction barrière des épidermes humains reconstruits par évaluation histologique desdits épidermes reconstruits humains, par coloration hématoxyline et éosine.*

La coloration hématoxyline et éosine des épidermes a été évaluée par l'attribution de « score » tel que décrit précédemment, et les résultats sont consignés dans le tableau 9 suivant :

**Tableau 9**

| | Score histologique |
|---|---|
| Epiderme humain reconstruit non traité (Contrôle) | 0<br>Pas de modification significative vs morphologie de référence |
| Epiderme humain colonisé avec *Staphylococcus epidermidis* + *Staphylococcus aureus* sans ajout de la composition ($C_{1A}$) | 2<br>Modification de la structure de l'épiderme viable avec plus d'espaces intercellulaires et des regroupements de cellules. |

(suite)

| Score histologique | |
|---|---|
| Epiderme humain reconstruit colonisé avec *Staphylococcus epidermidis* + *Staphylococcus aureus* et traité par la Composition ($C_{1A}$) 1% (v/v) (0,0136% d'extrait sec dû à la plante de la composition ($C_{1A}$)) | 1<br>Réduction des dommages, en particulier au niveau basal et du *stratum corneum* où une structure lamellaire plus compacte est observée |

Lorsque les épidermes humains reconstruits ont été colonisés avec *Staphylococcus. epidermidis* et *Staphylococcus aureus,* le score histologique a été évalué à un niveau de 2, témoignant d'une modification de la structure de l'épiderme viable avec plus d'espaces intercellulaires et des regroupements de cellules.

Lorsque les épidermes humains reconstruits ont été mis en contact avec la Composition ($C_{1A}$) en même temps que leur colonisation avec *Staphylococcus epidermidis* et *Staphylococcus aureus,* le score histologique a été évalué à un niveau de 1, témoignant d'une réduction des dommages, en particulier au niveau basal et du *stratum corneum* où une structure lamellaire plus compacte est observée.

Il en résulte que l'application sur la peau d'une composition comprenant la Composition ($C_{1A}$) permet de prévenir la dégradation de la cohésion tissulaire et par conséquent de la fonction barrière de l'épiderme face à une invasion de la flore transitoire.

Par ailleurs, dans le cadre de cette étude histologique, le profil de colonisation par les deux bactéries *(Staphylococcus epidermidis* et *Staphylococcus aureus*) a été évalué par microscopie électronique scannée (« Scanning electron microscopy » ou « SEM », Zeiss Sigma Electron Microscope).

Lorsque les épidermes humains reconstruits ont été colonisés par seulement *Staphylococcus epidermidis* la bactérie est présente de manière homogène à la surface de l'épiderme humain reconstruit, formant de grands agrégats et développant un biofilm caractérisé par des structures polysaccharidiques filamenteuses observées avec un grossissement de x10000 du microscope électronique .

Lorsque les épidermes humains reconstruits ont été colonisés par *Staphylococcus epidermidis* et *Staphylococcus aureus,* on observe l'apparition de plusieurs agrégats sphériques de *Staphylococcus aureus* à la surface de l'épiderme humain reconstruit, alors qu'un film de *Staphylococcus epidermidis* reste visible à la surface dudit épiderme.

On observe également la présence de grands agrégats de *Staphylococcus. Aureus,* avec un grossissement de x10000 du microscope électronique, formant une structure en trois dimension, indiquant ainsi un stade précoce de développement d'un biofilm à la surface de l'épiderme.

Après application sur la peau d'une composition comprenant la Composition ($C_{1A}$), les agrégats sphériques de *Staphylococcus aureus* ne sont plus présents, signifiant ainsi que l'ajout de la Composition ($C_{1A}$) permet de prévenir l'adhésion des bactéries à la surface de l'épiderme et la formation du biofilm de *Staphylococcus aureus.* Le biofilm de *Staphylococcus epidermidis* reste toujours observé à la surface de l'épiderme.

Ces observations montrent que la Composition ($C_{1A}$) permet de réduire l'adhésion et donc la formation de biofilms de bactéries opportunistes pathogènes, comme par exemple *Staphylococcus aureus,* sans altérer la présence de bactéries commensales telles que *Staphylococcus epidermidis.*

*B.1.3. Conclusions*

La combinaison de la mesure de la résistance électrique trans-épithéliale, et de l'évaluation histologique d'épidermes reconstruits humains, avant colonisation par une bactérie commensale de la flore cutanée, puis par une bactérie pathogène, constitue un modèle permettant d'étudier l'altération de la fonction barrière et l'équilibre du microbiote dudit épiderme, et l'incidence de traitements préalables avec des compositions ou des extraits ou des formulations complexes.

L'ensemble des résultats et des observations recueillis dans les sections B.1.2.1 et B.1.2.2 montre que la composition ($C_{1A}$) permet de prévenir la dégradation de la fonction barrière de l'épiderme de la peau humaine et par conséquent de prévenir l'apparition d'inflammation et de rougeurs, lorsqu'elle est colonisée par une bactérie commensale de la flore cutanée, puis par une bactérie pathogène.

**$B_2$) Mise en évidence de la prévention de l'effet de compositions selon l'invention sur l'inflammation induite en cas de déséquilibre du microbiote**

*B.2.1. Principe de la méthode*

L'effet anti-inflammatoire de compositions a été évalué sur des kératinocytes humains normaux en conditions mimant un déséquilibre du microbiote cutané par l'activation de 3 TLR (« Toll-Like Receptors »). Ces récepteurs sont activés par reconnaissance de motifs spécifiques des microbes et ont pour rôle d'alerter le système immunitaire afin d'activer les défenses de l'organisme. Dans ce modèle, le déséquilibre du microbiote a été modélisé avec différents stimuli mimant une infection cutanée :

- le TLR-5 a été activé par de la flagelline (protéine principale du filament flagellaire, présent sur presque toutes les bactéries)
- le TLR-2 par du zymosan (complexe glycoprotéique extrait des membranes de levures)
- le TLR-3 par du poly(I:C) (un analogue synthétique de l'ARN double-brin retrouvé dans des virus)

L'effet des compositions testées a été évalué sur sa capacité à moduler la production d'IL-8 et d'hBD2 induite par ces stimuli en utilisant des kits « ELISA » spécifiques. La concentration en protéines totales a également été évaluée afin de normaliser la production de cytokine (IL-8) et de peptide anti-microbien (hBD2).

*Eléments statistiques :*

Les valeurs sont exprimées en moyennes +/- sem [standard error of the mean ou erreur type de la moyenne = écart-type / racine (nombre de valeurs)].
Pour chaque traitement, on a calculé :

% protection = 100 x [ moyenne (cellules + traitement) - moyenne (cellules stimulées) ] / [ moyenne (cellules non stimulées) - moyenne (cellules stimulées) ]

L'analyse statistique des résultats a été menée à l'aide d'un test de Student bilatéral avec un seuil de significativité fixé à 5%, en comparant les séries de valeurs deux à deux.
On considérera qu'une différence entre l'efficacité de deux produits est :

- Significative si $p < 0,05$ ;
- Dite « à la limite de significativité » si $0,05 \le p < 0,1$ ;
- Et non significative si $p > 0,1$.

*B.2.2. Résultats obtenus*

Dans les trois tableaux qui suivent : Pour cellules stimulées : *** p<0,001 vs cellules non stimulées. Pour cellules traitées et stimulées : *** p<0,001 vs cellules stimulées.
Les résultats obtenus suite à l'activation de TLR-5 par la flagelline, les résultats sont consignés dans le tableau 10 ci-dessous :

**Tableau 10**

| | IL-8 (pg/mg protéines) | hBD2 (pg/mg protéines) |
|---|---|---|
| Contrôle (cellules non stimulées) | 22 +/- 1 | 39 +/- 5 |
| Cellules stimulées avec de la flagelline | 906 +/- 42*** | 629 +/- 65*** |
| Cellules stimulées avec de la flagelline + référence positive (IKK inhibitor X 10 µM) | 304 +/- 9 68%*** | 90 +/- 11 91%*** |
| Cellules stimulées avec de la flagelline + composition ($C_{1A}$) à 0,074% (v/v) | 363 +/- 29 61%*** | 126 +/- 7 85% *** |

Les résultats obtenus suite à l'activation TLR-2 par le zymosan, les résultats sont consignés dans le tableau 11 ci-dessous :

**Tableau 11**

| | IL-8 (pg/mg protéines) | hBD2 (pg/mg protéines) |
|---|---|---|
| Contrôle (cellules non stimulées) | 21 +/- 1 | 53 +/- 9 |
| Cellules stimulées avec du zymosan | 1421 +/- 31*** | 558 +/- 33*** |
| Cellules stimulées avec du zymosan + référence positive (IKK inhibitor X 10 $\mu$M) | 373 +/- 8 75%*** | 55 +/- 9 100%*** |
| Cellules stimulées avec du zymosan + composition ($C_{1A}$) à 0,074% | 661 +/- 28 54%*** | 267 +/- 8 58%*** |

Les résultats obtenus suite à l'activation de TLR-3 par le poly(I:C), les résultats sont consignés dans le tableau 12 ci-dessous :

**Tableau 12**

| | IL-8 (pg/mg protéines) | hBD2 (pg/mg protéines) |
|---|---|---|
| Contrôle (cellules non stimulées) | 22 +/- 2 | 40 +/-45 |
| Cellules stimulées avec du poly(I:C) | 15109 +/- 147*** | 480 +/- 26*** |
| Cellules stimulées avec du poly(I:C) + référence positive (Bafilomycine 100 nM) | < 19 100%*** | 17 +/- 2 100%*** |
| Cellules stimulées avec du poly(I:C) + composition ($C_{1A}$) à 0,074% | 3986 +/- 520 74%*** | 105 +/- 20 85%*** |
| Cellules stimulées avec du poly(I:C) + composition ($C_{1A}$) 3,13 x $10^{-4}$ % extrait sec | 875 +/- 40 94%*** | 22 +/- 3 100%*** |

*B.2.3. Analyses et conclusions*

Lorsque le TLR-5 est activé par la flagelline, et lorsque on traite les cellules par 0,074% de la composition ($C_{1A}$) selon l'invention, on observe une augmentation de la production d'IL-8 de 61% par rapport à la production d'IL-8 lorsque les cellules ne sont pas traitées. De même, lorsque on traite les cellules par la composition ($C_{1A}$) selon l'invention, on observe une augmentation de la production de hBD2 de 85% par rapport à la production de hBD2 lorsque les cellules ne sont pas traitées.

Lorsque le TLR-2 est activé par le zymosan, et lorsque on traite les cellules par 0,074% de la composition ($C_{1A}$) selon l'invention, on observe une augmentation de la production d'IL-8 de 54% par rapport à la production d'IL-8 lorsque les cellules ne sont pas traitées. De même, lorsque on traite les cellules par la composition ($C_{1A}$) selon l'invention, on observe une augmentation de la production de hBD2 de 58% par rapport à la production de hBD2 lorsque les cellules ne sont pas traitées.

Lorsque le TLR-3 est activé par le poly(I:C), et lorsque on traite les cellules par 0,074% de la composition ($C_{1A}$) selon l'invention, on observe une augmentation de la production d'IL-8 de 74% par rapport à la production d'IL-8 lorsque les cellules ne sont pas traitées. De même, lorsque on traite les cellules par la composition ($C_{1A}$) selon l'invention, on observe une augmentation de la production de hBD2 de 85% par rapport à la production de hBD2 lorsque les cellules ne sont pas traitées.

Lorsque les TLR-2, TLR-3 et TLR-5 sont activés, et notamment par simulation d'une infection (bactérie, levure, virus), l'association de la composition ($C_{1A}$) selon l'invention permet de réduire la surproduction de la cytokine IL-8 et du peptide anti-microbien hBD2, ce qui montre sa capacité à réduire l'inflammation induite par le déséquilibre du microbiote. Par conséquent, l'association de la composition ($C_{1A}$) selon l'invention permettant de réduire la surproduction de la cytokine IL-8 et du peptide anti-microbien hBD2, agit sur la limitation du phénomène d'inflammation cutanée ou du cuir chevelu, et par conséquent de diminuer les effets inesthétiques liés à l'acné.

**$B_3$) Mise en évidence de l'effet de la composition selon l'invention sur la prévention des rougeurs et des sensations de brûlure et/ou de démangeaisons suite à l'induction d'irritation physique ou chimique pour des peaux réactives**

*B.3.1. Principe de la méthode*

La méthode consiste à évaluer l'effet des compositions testées sur l'équilibre du microbiote et de la fonction barrière, pouvait contribuer à apaiser les peaux réactives activées par des stress mécanique ou chimique.

Une population de 20 femmes âgées de 18 à 65 ans, ayant la peau sensible (sur la base de leur déclaration et d'un test de stinging $\geq 4$), ont été recrutées.

Le "stinging test" est utilisé pour identifier les personnes ayant une réactivité cutanée particulière au niveau du visage. Il consiste à appliquer un stress chimique (cinq dépôts d'une solution d'acide lactique à 10% massique au niveau du sillon naso-génien, comparativement à du sérum physiologique appliqué simultanément de l'autre côté. Le sujet évalue les sensations de brûlure et de picotement après 15 secondes, puis 30 secondes, 2 minutes et 5 minutes, suivant l'application, selon la grille d'évaluation suivante : 0 = aucune sensation ; 1 = sensation légère ; 2 = sensation modérée ; 3 = sensation intense. Pour chaque sujet, la somme des scores est calculée et si elle est supérieure ou égale à 4, le sujet est qualifié de sujet à peau réactive.

La population de 20 personnes sélectionnées a appliqué la formule contenant la composition (C1A) selon l'invention et la formule placebo en hémi-visage, 2 fois par jour, pendant 14 jours. Après 14 jours d'application, la population de sujets a subi un stress mécanique et un stress chimique pour évaluer l'effet apaisant des compositions testées :

- La réactivité cutanée a tout d'abord été évaluée par la mesure de la couleur rouge de la peau à l'aide d'un chromamètre qui mesure le paramètre "a". Le stress mécanique a ensuite été réalisé sur les joues par cinq « strippings » (stripage/pelage) successifs qui enlèvent les premières couches superficielles de la peau. Puis les produits ont été appliqués (un de chaque côté du visage) et la réactivité cutanée a été de nouveau mesurée par chromamétrie par mesure du paramètre "a" 30 minutes après la première mesure. La différence sur le paramètre " $\Delta a$" avant et 30 minutes après a été calculé comme suit :

  Pour chaque sujet (i), $\Delta a_i$ = valeur du paramètre (a) 30 minutes après la première mesure - valeur du paramètre (a) avant application.
  Pour l'ensemble de la population, $\Delta a_{moy} = (\Sigma\ \Delta a_i)$ /nombre de sujets

- Le stress chimique a été réalisé par 5 applications d'une solution d'acide lactique 10% au niveau des sillons naso-géniens. Chaque sujet (i) a évalué (même échelle que précédemment) les sensations de brûlure et de picotements après 15 secondes suivant l'application de l'acide lactique (noté «$S_{i0}$ »), puis les produits ont été appliqués (un de chaque côté du visage), et à nouveau chaque sujet (i) a évalué les sensations de brûlure et de picotements à 30 secondes (noté «$S_{i30s}$ »), 2 minutes (noté «$S_{i2min}$ »), et 5 minutes (noté «$S_{i5min}$ »).

  Pour chaque sujet (i), on calcule $\Delta S_i = (S_{i30s} + S_{i2min} + S_{i5min}) - S_{i0}$

  Pour l'ensemble de la population, on calcule $\Delta S_{moy} = (\Sigma\ S_i)$/nombre de sujets

*B.3.2. Résultats obtenus*

• Evaluation des rougeurs induites par un stress mécanique ($\Delta a_{moy}$)

Les paramètres $\Delta a_{moy}$ mesurés sont les suivants :

$\Delta a_{moy}$ = 1,41 pour le groupe pour lequel le paramètre « a » a été mesuré sur les hémi-visages sur lesquels a été appliquée la formule placebo.
$\Delta a_{moy}$ = 0,57 pour le groupe pour lequel le paramètre « a » a été mesuré sur les hémi-visages sur lesquels a été appliquée la formule contenant la composition ($C_{1A}$)

L'application de la formule contenant la composition ($C_{1A}$) permet donc de limiter de 60% le paramètre $\Delta a_{moy}$, et donc de limiter le phénomène des rougeurs liées à l'inflammation cutanée induite par un stress mécanique.

• Evaluation des sensations de brûlure et de picotements induites par un stress chimique ($\Delta S_{moy}$)

$\Delta S_{moy}$ = 1 pour le groupe pour lequel le critère $\Delta S_{moy}$ a été mesuré sur les hémi-visages sur lesquels a été appliquée la

formule placebo.
$\Delta S_{moy}$ = 0,8 pour le groupe pour lequel le critère $\Delta S_{moy}$ a été mesuré sur les hémi-visages sur lesquels a été appliquée la formule contenant la composition ($C_{1A}$).

L'application de la formule contenant la composition ($C_{1A}$) permet donc de réduire le critère d'évaluation liée au stress chimique $\Delta S$ de 20%, donc de diminuer le phénomène des sensations de brûlures et de picotements de la peau humaine induit par un stress chimique.

**$B_4$) Mise en évidence de l'effet de la composition ($C_{1A}$) sur l'inflammation induite en cas de déséquilibre du microbiote (activité anti-lipase)**

*B.4.1. Principe de la méthode*

Il s'agit d'étudier la capacité d'une composition à provoquer et réguler l'activité de l'enzyme lipase par une méthode *in tubo,* ladite enzyme ayant une action inflammatoire.
La lipase présente la capacité de transformer le 1,2-diglycéride, incolore, en glycérol qui est d'une couleur rose.
Les échantillons à l'essai d'évaluation sont mis *in tubo* en présence de lipase et de 1,2-diglycéride et l'absorbance des échantillons est mesurée au spectrophotomètre à la longueur d'onde 570 nm dès la fin de leur préparation, puis au bout de 60 minutes d'incubation à 37°C dans les mêmes conditions spectrales. Grâce à une gamme de glycérol, l'activité lipase des échantillons peut être calculée, ainsi que le pourcentage d'inhibition selon les formules suivantes :

Activité lipase = (quantité de glycérol formée entre 0 et 60 minutes) / ( 60 minutes x volume échantillon )

Pourcentage d'inhibition = 100 x [ (activité lipase du groupe témoin) - (activité lipase du produit à l'essai) ] / (activité lipase du groupe témoin)

*Eléments statistiques :*

Les valeurs sont exprimées en moyennes +/- écart-type.
L'analyse statistique des résultats a été menée à l'aide d'un test de Student bilatéral avec un seuil de significativité fixé à 5%, en comparant les séries de valeurs deux à deux.
On considérera qu'une différence entre l'efficacité de deux produits est :

- Significative si $p < 0,05$ ;
- Dite « à la limite de significativité » si $0,05 \leq p < 0,1$ ;
- Et non significative si $p > 0,1$.

*B.4.2. Résultats obtenus*

Les résultats obtenus sont consignés dans le tableau 13 ci-dessous (*** p<0,001 vs témoin).

**Tableau 13**

| Produits testés | Activité lipase (mU/mL) | % d'inhibition de la lipase |
|---|---|---|
| Témoin | 4,21 +/- 0,02 | 0 |
| Référence positive (Vitamine C) | 2,11 +/- 0,01 | 50*** |
| Composition ($C_{1A}$) à 1% massique | 0,92 +/- 0,01 | 78*** |
| Propylène Glycol 70% à 1% | 6,40 +/- 0,05 | - |

*B.4. 3. Analyse des résultats*

Les mesures consignées dans le tableau 13 montrent que le traitement par la composition selon l'invention ($C_{A1}$) réduit de façon très significative l'activité de la lipase, puisque le pourcentage d'inhibition mesuré est de 78%.
La composition selon l'invention ($C_{A1}$) permet de limiter l'activité de la lipase et par conséquent de diminuer les

effets inesthétiques de l'inflammation de la peau liée à l'activité de ladite lipase.

**$B_5$) Conclusions générales sur les évaluations biologiques mettant en œuvre la composition selon l'invention ($C_{A1}$).**

Les évaluations expérimentales de cette section ont démontré que la composition ($C_{1A}$) selon l'invention, permet de limiter la formation d'un biofilm de bactéries pathogènes (en l'occurrence Staphylococcus aureus), sans atteindre cependant sa viabilité ni celle de bactéries commensales (comme par exemple Staphylococcus epidermidis).

De même, il a été établi que

- lorsque les TLR-2, TLR-3 et TLR-5 sont activés, et notamment par simulation d'une infection (bactérie, levure, virus), l'association de la composition ($C_{1A}$) selon l'invention permet de réduire la surproduction de la cytokine IL-8 et du peptide anti-microbien hBD2, ce qui montre sa capacité à réduire l'inflammation induite par le déséquilibre du microbiote,
- L'application de la formule contenant la composition ($C_{1A}$) permet donc de diminuer le phénomène des rougeurs liées à l'inflammation cutanée ;
- L'application de la formule contenant la composition ($C_{1A}$) permet de diminuer le phénomène des sensations de brûlures et de picotement de la peau humaine.

Il en résulte que la composition selon l'invention ($C_{A1}$) peut être utilisée dans le but d'empêcher ou de ralentir l'apparition des signes inesthétiques liés à l'inflammation de la peau et/ou du cuir chevelu.

**C) Formulations**

Dans les formules suivantes, les pourcentages sont exprimés en poids de la formulation.

**C.1 Fluide démaquillant visage**

Formule

| | |
|---|---|
| **Composition** ($C_{1A}$) | 10,00% |
| Méthyl paraben | 0,15% |
| Phenoxyethanol | 0,80% |
| SEPICALM™ S | 1,00% |
| Parfum/Fragrance | 0,10% |
| Eau | qs. 100,00% |

Mode opératoire : Mélanger les différents ingrédients dans l'eau sous agitation magnétique dans l'ordre indiqué, et ajuster le pH aux alentours de 7.

**C.2 Shampoing cheveux et corps pour enfants**

Formule

| | | |
|---|---|---|
| A | Composition ($C_{1A}$) | 15,00% |
| | PROTEOL™APL | 5,00% |
| | SEPICIDE™HB | 0,50% |
| | Parfum/Fragrance | 0,10% |
| B | Eau | 20,00% |
| | CAPIGEL™98 | 3,50% |
| C | Eau | Q.S. 100,00% |

(suite)

| | |
|---|---|
| SEPICIDE™CI | 0,30% |
| Colorant | Q.S |
| Soude | Q.S. pH = 7,2 |

Mode opératoire : Mélanger la composition ($E_4$) avec le PROTEOL™APL, et le SEPICIDE™HB (Phase A). Diluer le CAPIGEL™98 dans une partie de l'eau et l'ajouter à la phase A précédemment obtenue (Phase B). Ajouter le reste d'eau à la phase B, puis le SEPICIDE™CI et le colorant. Ajuster le pH du mélange à 7,2 environ avec de la soude.

**C.3 Lingettes démaquillantes pour les yeux**

Formule

| | | |
|---|---|---|
| A | Composition ($C_{1A}$) | 3,00% |
| B | SEPICIDE™HB2 | 0,50% |
| C | SEPICALM™ VG | 0,50% |
| | Parfum/Fragrance | 0,05% |
| D | Eau | Q.S. 100,00% |

Mode opératoire : Mélanger les ingrédients de la phase B ainsi que ceux de la phase C dans la phase A jusqu'à obtenir la limpidité de la solution. Ajouter la phase D.

**C.4 Gel moussant doux**

Formule

| | | |
|---|---|---|
| A | Composition ($C_{1A}$) | 8,50% |
| | PROTEOL™ APL | 3,00% |
| | EUXYL ™ PE9010 | 1,00% |
| | Parfum/Fragrance | 0,10% |
| B | Eau | Q.S. 100,00% |
| | Acide lactique | Q.S. pH = 6,0 |

Mode opératoire : Solubiliser le parfum et le conservateur EUXYL™ PE 9010 dans le mélange composé de la composition $E_4$ et du PROTEOL™ APL (phase A). Ajouter l'eau et régler le pH à environ 6,0 avec de l'acide lactique.

**C.5 Shampoing à usage fréquent**

Formule

| | | |
|---|---|---|
| A | Composition ($C_{1A}$) | 12,80% |
| | PROTEOL™ OAT | 5,00% |
| | EUXYL ™ PE 9010 | 1,00% |
| | Parfum/Fragrance | 0,30% |
| | Eau Q.S. | 100,00% |
| B | MONTALINE™C40 | 8,50% |
| | Acide lactique | Q.S. pH = 6,0 |

Mode opératoire : mélanger tous les ingrédients de la phase A et, après homogénéisation, ajouter la MONTALINE™C40 et ajuster le pH à environ 6,0 à l'aide de l'acide lactique.

**C.6 Shampoing ultra-doux pour bébé**

Formule

| | | |
|---|---|---|
| A | Composition ($C_{1A}$) | 10,00% |
| | AMISOFT™CS-11 | 4,00% |
| | Parfum/Fragrance | 0,10% |
| | SEPICIDE™HB | 0,30% |
| | SEPICIDE™CI | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : Mélanger tous les ingrédients de la phase A dans l'ordre indiqué jusqu'à l'obtention d'une phase A limpide. De façon séparée, ajouter le CAPIGEL™98 dans l'eau, puis ajouter cette phase B ainsi préparée dans la phase A et ajuster le pH à 7,2 à l'aide de la trométhamine.

**C.7 Lait de toilette pour bébé**

Formule

| | | | |
|---|---|---|---|
| A | SIMULSOL™165 | 2,00% | |
| | MONTANOV™202 | 1,00% | |
| | LANOL™99 | | 3,00% |
| | Dimethicone | | 1,00% |
| | Isohexadecane | | 3,00% |
| B | Eau | | Q.S. 100,00% |
| C | SEPIPLUS™400 | | 0,30% |
| D | Composition ($C_{1A}$) | | 6,35% |
| E | SEPICIDE™HB | | 0,30% |
| | DMDM Hydantoin | | 0,20% |
| | Parfum/Fragrance | | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

**C.8 Lotion poudrée nettoyante pour peaux sensibles**

Formule

| | | |
|---|---|---|
| A | LIPACIDE™C8G | 0,95% |

(suite)

| | | |
|---|---|---|
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | Composition ($C_{1A}$) | 1,80% |
| | Parfum/Fragrance | 0,10% |
| C | SEPICALM™S | 0,28% |
| | Eau | Q.S. 100,00% |
| | Acide lactique | Q.S. pH = 5,2 |
| D | MICROPEARL™M310 | 5,00% |

Mode opératoire : Solubiliser les ingrédients de la phase A dans l'eau à 80°C. Solubiliser séparément le parfum dans la composition ($E_4$) pour préparer la phase B. Ajouter la phase A refroidie sur la phase B, puis introduire le SEPICALM™S et le complément d'eau. Vérifier le pH final et éventuellement l'ajuster à environ 5,2. Ajouter alors le MICROPEARL™ M310.

**C.9 Gel douche enfants**

Formule

| | | |
|---|---|---|
| A | Eau | 56,06% |
| | SEPIMAX™Zen | 3,00% |
| | SEPIPLUS™S | 0,80% |
| B | PROTEOL™OAT | 20,80% |
| | ORAMIX™NS 10 | 9,30% |
| | AMONYL™265 BA | 5,10% |
| C | Composition ($C_{1A}$) | 2,00% |
| | Glycéryl Glucoside | 1,00% |
| | Phenoxyéthanol & Ethylhexyl Glycérine | 1,00% |
| | Parfum/Fragrance | 0,90% |
| | Colorant | 0,04% |

Mode opératoire : disperser le SEPIMAX™ZEN dans l'eau et agiter à l'aide d'un agitateur mécanique muni d'une défloculeuse, d'une contrehélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogénéiser et ajouter individuellement les additifs de la phase C. Ajuster le pH à 6.0 - 6.5.

**C.10 BB Crème**

Formule

| | | |
|---|---|---|
| A | EASYNOV™ | 2,30% |
| | LANOL™ 99 | 1,00% |
| | SEPIMAT™ H10W | 1,00% |

(suite)

| | | |
|---|---|---|
| | Ethylhexyl methoxycinnamate | 5,00% |
| B | Cyclométhicone | 6,00% |
| | Triethoxycaprylsilane & Alumina-silane & Titanium Oxide | 8,00% |
| | Iron Oxide red & Triethoxycaprylsilane | 0,24% |
| | Iron Oxide yellow & Triethoxycaprylsilane | 0,66% |
| | Iron Oxide black & Triethoxycaprylsilane | 0,09% |
| | Parfum/Fragrance | 0,10% |
| C | Eau | qs 100% |
| | SEPINOV™EMT10 | 1,20% |
| D | Composition ($C_{1A}$) | 2,00% |
| | SEPITONIC™M3 | 1,00% |
| | Phenoxyéthanol & Ethylhexyl Glycérine | 1,00% |

Mode opératoire : Préparer la phase B par mélange des différents ingrédients et homogénéiser à l'aide d'un mélangeur muni d'un système de rotor-sator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-sator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase 5 et agiter à une vitesse de 50 tours par minute pendant 25 minutes.

**C.11 Spray Solaire haute Protection SPH supérieur à 30**

Formule

| | | |
|---|---|---|
| A | MONTANOV™L | 1,00% |
| | MONTANOV™82 | 1,00% |
| | C12-15 Alkylbenzoate | 17,00% |
| | Dimethicone | 3,00% |
| | Octocrylène | 6,00% |
| | Ethylhexyl methoxycinnamate | 6,00% |
| | Bis-ethylhexyloxyphenol Méthoxypenyl Triazine | 3,00% |
| | Tocophérol | 0,05% |
| B | Eau | qs 100% |
| C | SIMULGEL™INS 100 | 0,50% |
| | Cyclodiméthicone | 5,00% |
| D | Composition ($C_{1A}$) | 3,00% |
| | Phenoxyéthanol & Ethylhexyl Glycérine | 1,00% |
| | Parfum/Fragrance | 0,20% |
| E | Methylene Bis-Benzotriazolyl Tetraméthylbutylphénol | 10,00% |

(suite)

| | |
|---|---|
| Acide citrique 25% | qs pH = 5 |

SEPICALM™S : Mélange de N-cocoyl aminoacides, de sarcosine, d'aspartate de potassium et d'aspartate de magnésium tel que décrit dans WO 98/09611 .
PROTEOL™APL : Mélange de sels de sodium de N-cocoyl aminoacides, obtenus par acylation des acides aminés caractéristiques du jus de pomme ;
SEPICIDE™HB : Mélange de phénoxyéthanol, de méthylparaben, d'éthylparaben, de propylparaben et de butylparaben, est un agent conservateur .
CAPIGEL™98 : Copolymère d'acrylates ;
SEPICIDE™CI : Imidazoline urée, est un agent conservateur ;
SEPICIDE™HB : Mélange de phénoxyéthanol, de méthylparaben, d'éthylparaben, de propylparaben, de butylparaben et d'isobutylparaben, est un agent conservateur ;
SEPICALM™VG : Mélange de N-palmitoyl proline sous forme de sel de sodium et d'extrait de fleur de Nymphea Alba ;
EUXYL™PE9010 : Mélange de phénoxyéthanol et d'ethyl hexyl glycérine ;
PROTEOL™OAT : Mélange de N-lauryl aminoacides obtenus par hydrolyse totale de protéine d'avoine tel que décrit dans WO 94/26694 ;
MONTALINE™C40 : Sel de chlorure de Cocamidopropyl betaïnamide de Monoéthanolamine.
AMISOFT™CS-11 : Sel de sodium de N-cocoyl glutamate ;
SIMULSOL™165 : Mélange de stéarate de PEG-100 et de stéarate de glycérol ;
MONTANOV™202 (alcool arachydilique, alcool béhénique et arachidyl glucoside), est une composition auto-émulsionnable telle que celles décrites dans EP 0 977 626 ;
LANOL™99 : Isononoate d'isononyle ;
SEPIPLUS™400 : Latex inverse auto-inversible de polyacrylates dans le polyisobutene et comportant du polysorbate 20, tel que décrit dans WO2005/040230 ;
LIPACIDE™C8G : Capryloyl glycine commercialisé par la société SEPPIC ;
MICROPEARL™M310 : Polymère polyméthylméthacrylate réticulé se présentant sous forme de poudre et utilisé comme modificateur de texture ;
SEPIMAX™Zen (nom INCI : Polyacrylate Crosspolymer-6) : Polymère épaississant se présentant sous la forme d'une poudre ;
SEPIPLUS™S (nom INCI : Hydroxyethyl Acrylate / Sodium Acryloyldimethyl Taurate Copolymer & Polyisobutene & PEG-7 Trimethylolpropane Cononut Ether) : Latex inverse auto-inversible ;
AMONYL™265 BA (nom INCI : Cocobétaïne) : Agent tensioactif amphotère moussant ;
SEPINOV™EMT10 (nom INCI : Hydroxyethyl Acrylate / Sodium Acryloyldimethyl Taurate Copolymer) : Copolymère épaississant se présentant sous la forme d'une poudre ;
EASYNOV™ (nom INCI : Octyldodecanol and Octyldodecyl Xyloside and PEG-30 Dipolyhydroxystearate) : Agent émulsionnant à tendance lipophile ;
SEPIMAT™H10 FW (nom INCI :Methyl Methacrylate Crosspolymer and Squalane) : Polymère utilisé comme agent de texture ;
SEPITONIC™M3 (nom INCI : Magnesium Aspartate and Zinc Gluconate and Copper Gluconate) : Mélange utilisé comme agent antiradicalaire et énergisant pour les cellules ;
MONTANOV™L (nom INCI : C14-22 Alcohols and C12-20 Alkylglucoside) : Agent émulsionnant ;
MONTANOV™82 (nom INCI : Cetearyl Alcohol and Coco-glucoside) : Agent émulsionnant ;
SIMULGEL™INS100 (nom INCI :Hydroxyethyl Acrylate/Sodium Acryloydimethyl Taurate Copolymer and isohexadecane and Polysorbate 60) : Agent épaississant polymèrique ;

## Revendications

**1.** Composition (C1) comprenant pour 100% de sa masse :

a) - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;
b) - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

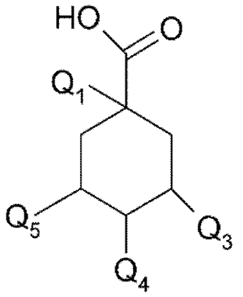

(I),

dans laquelle $Q_1$, $Q_2$, $Q_3$, $Q_4$ et $Q_5$ représentent indépendamment les uns des autres, le radical hydroxyle ou un ses sels ou un radical choisi parmi :

**(i)** - Le radical caféoyle de formule (II) :

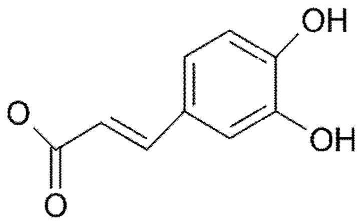

(II)

**(ii)** - Le radical maloyle de formule (IIIa) ou (IIIb) :

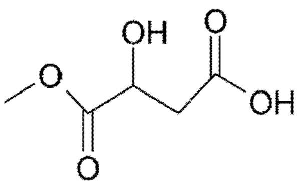

(IIIa)

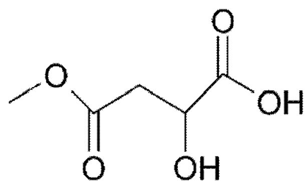

(IIIb) ;

**(iii)** - Le radical caféoyl maloyle de formule (IVa) ou (IVb) :

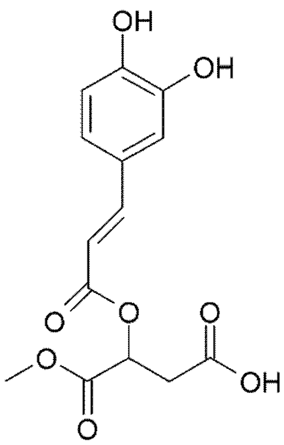

(IVa)

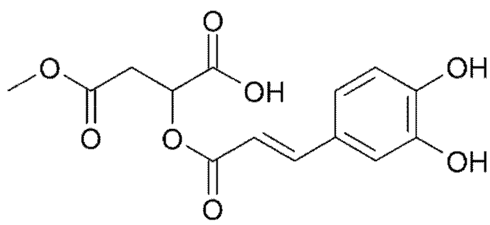

(IVb)

**(iv)** - Le radical maloyl caféoyle de formule (Va), (Vb), (Vc) ou (Vd),

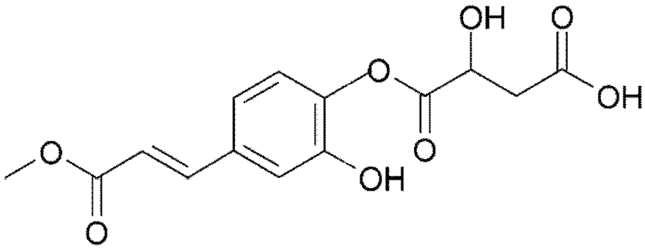

(Va)

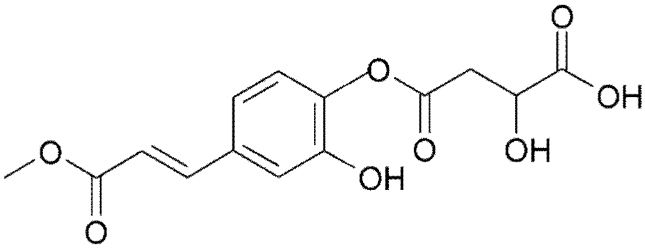

(Vb)

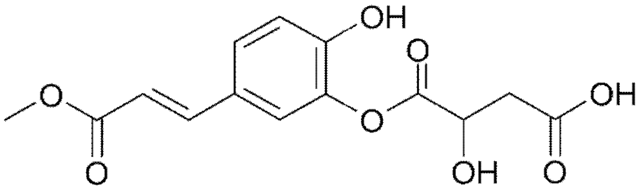

(Vc),

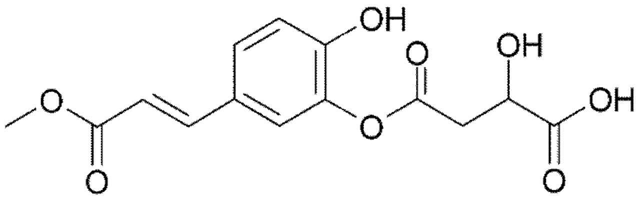

(Vd)

étant entendu qu'au moins un de ces radicaux $Q_1$, $Q_2$, $Q_3$, $Q_4$ et $Q_5$ ne représente ni le radical -OH ; ni un de ses sels ;
ladite composition (ES) comprenant au moins :

- Au moins un composé de formule (Ia) correspondant à la formule (I) pour laquelle $Q_1$ représente le radical maloyle de formule (IIIa) ou de formule (IIIb) et $Q_3$ et $Q_4$ et $Q_5$ identiques, représentent chacun le radical caféoyle de formule (II) ;
- **Un composé** de formule (Ib) correspondant à la formule (I) pour laquelle $Q_1$ représente le radical caféoylmaloyle de formule (IVa) ou de formule (IVb), $Q_3$ et $Q_5$ représentent chacun le radical caféoyle de formule (II) et $Q_4$ représente le radical hydroxyle, et
- Au moins un composé de formule (Ic) choisi parmi :

  - Le composé de formule ($Ic_1$) correspondant à la formule (I) pour laquelle $Q_1$ et $Q_5$ représentent chacun le radical caféoyle de formule (II), $Q_3$ représente le radical hydroxyle et $Q_4$ représente le radical caféoylmaloyle de formule (IVa) ou de formule (IVb) ; et

    - Le composé de formule ($Ic_2$) correspondant à la formule (I) pour laquelle $Q_1$ et $Q_4$ représentent le radical caféoyle de formule (II), $Q_3$ représente le radical hydroxyle et $Q_5$ représente le radical caféoylmaloyle de formule (IVa) ou de formule (IVb) ; et

- **c)** - De 20,0% massique à 35,0% massique d'eau,
pour empêcher ou ralentir l'apparition des signes inesthétiques liés à l'inflammation de la peau et/ou du

cuir chevelu chez l'être humain, ou bien de les éliminer

**2.** Composition selon la revendication 1, pour laquelle la composition ($C_1$) comprend pour 100% de sa masse :

- de 60,0% massique à 75,0% massique de 1,2-propanediol,
- de 0,1% massique à 2,0% massique d'une composition (ES) comprenant une quantité massique $x_1$, exprimée en équivalent massique de l'acide 1-O-(2-caféoyl)maloyl-3,5-O-dicaféoyl quinique, supérieure ou égale à 200 mg/ d'au moins le composé de formule (Ia), et d'au moins le composé de formule (Ib), et d'au moins le composé de formule (Ic)
- de 20,0% massique à 35,0% massique d'eau.

**3.** Composition ($C_2$) comprenant au moins un excipient cosmétiquement acceptable (E) et une composition ($C_1$) selon la revendication 2.

**4.** Composition selon la revendication 3, **caractérisée en ce qu'**elle est à usage topique.

**5.** Composition ($C_1$) telle que définie à la revendication 1 pour son utilisation dans une méthode de traitement thérapeutique visant à diminuer et/ou éliminer les picotements et/ou les fourmillements et/ou les démangeaisons et/ou les échauffements et/ou les rougeurs et/ou l'inconfort cutané et/ou les tiraillements de la peau provoqués par l'inflammation de la peau humaine et/ou le cuir chevelu.

**6.** Composition ($C_1$) selon la revendication 5, **caractérisée en ce que** les picotements et/ou les fourmillements et/ou les démangeaisons et/ou les échauffements et/ou les rougeurs et/ou l'inconfort cutané et/ou les tiraillements accompagnent les pathologies cutanées comme l'urticaire, les dermatites eczémateuses, la rosacée, le psoriasis, l'herpès, les photodermatoses, la dermatite atopique, la dermatite de contact, le lichen, les prurigos, les maladies prurigineuses, les fibroses, les troubles de la maturation du collagène, la sclérodermie, l'eczéma.

## Patentansprüche

**1.** Zusammensetzung (C1), umfassend für 100 % ihrer Masse:

a) - 60,0 Masse-% bis 75,0 Masse-% eines organischen Lösungsmittels ($SO_1$), ausgewählt aus 1,2-Propandiol, 1,3-Propandiol, 1,4-Butandiol, 1,3-Butandiol, 1,2-Butandiol, 2-Methyl-2,4-pentandiol, 1,6-Hexandiol, 1,8-Octandiol oder einer Mischung dieser Verbindungen;
b) - 0,1 Masse-% bis 2,0 Masse-% einer Zusammensetzung (ES), umfassend eine Massenmenge $x_1$, ausgedrückt als Massenäquivalent von 1-O-(2-Caffeoyl)maloyl-3,5-O-dicaffeoylchinasäure, die größer oder gleich 200 mg/g mindestens einer Verbindung der allgemeinen Formel (I) ist:

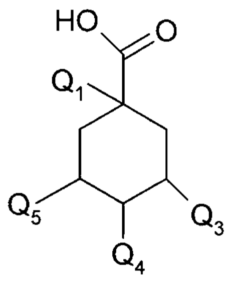

(I),

wobei $Q_1$, $Q_2$, $Q_3$, $Q_4$ und $Q_5$ unabhängig voneinander den Hydroxylrest oder eines seiner Salze oder einen Rest ausgewählt aus:

(i) - dem Caffeoylrest der Formel (II):

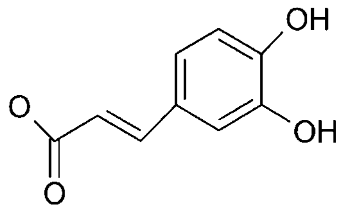

(II)

(ii) - dem Maloylrest der Formel (IIIa) oder (IIIb):

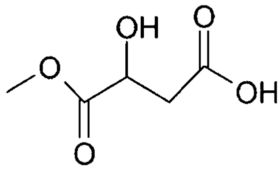

(IIIa)

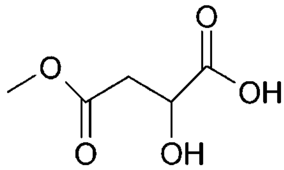

(IIIb),

(iii) - dem Caffeoylmaloylrest der Formel (IVa) oder (IVb):

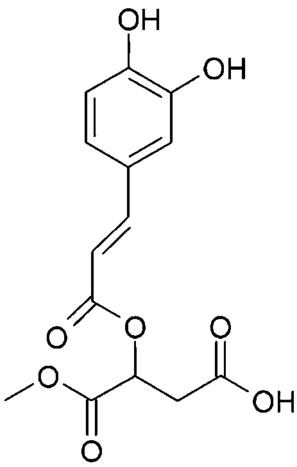

(IVa)

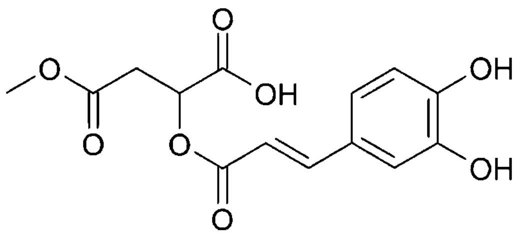

(IVb)

(iv) - dem Maloylcaffeoylrest der Formel (Va), (Vb), (Vc) oder (Vd) darstellen,

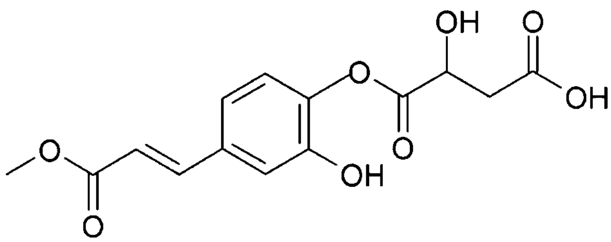

(Va)

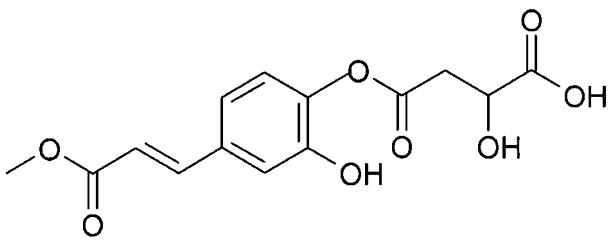

(Vb)

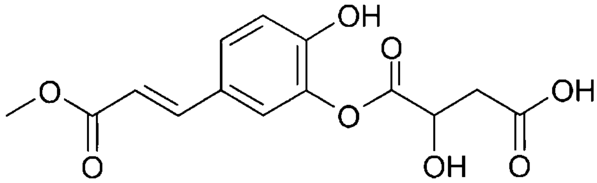

(Vc),

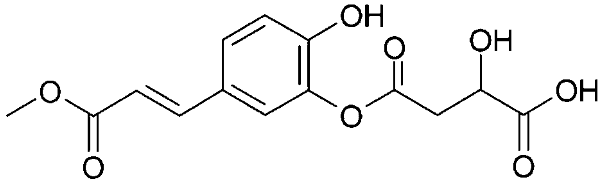

(Vd)

wobei es sich versteht, dass mindestens einer dieser Reste $Q_1$, $Q_2$, $Q_3$, $Q_4$ und $Q_5$ weder den - OH-Rest noch eines seiner Salze darstellt;
wobei die Zusammensetzung (ES) mindestens umfasst:

- Mindestens eine Verbindung der Formel (Ia), die der Formel (I) entspricht, bei der $Q_1$ den Maloylrest der Formel (IIIa) oder der Formel (IIIb) darstellt und $Q_3$ und $Q_4$ und $Q_5$, die identisch sind, jeweils den Caffeoylrest der Formel (II) darstellen;
- Eine Verbindung der Formel (Ib), die der Formel (I) entspricht, bei der $Q_1$ den Caffeoylmaloylrest der Formel (IVa) oder der Formel (IVb) darstellt, $Q_3$ und $Q_5$ jeweils den Caffeoylrest der Formel (II) darstellen und $Q_4$ den Hydroxylrest darstellt, und
- Mindestens eine Verbindung der Formel (Ic), ausgewählt aus:

  - der Verbindung der Formel ($Ic_1$), die der Formel (I) entspricht, bei der $Q_1$ und $Q_5$ jeweils den Caffeoylrest der Formel (II) darstellen, $Q_3$ den Hydroxylrest darstellt und $Q_4$ den Caffeoylmaloylrest der Formel (IVa) oder der Formel (IVb) darstellt; und
  - der Verbindung der Formel ($Ic_2$), die der Formel (I) entspricht, bei der $Q_1$ und $Q_4$ jeweils den Caffeoylrest der Formel (II) darstellen, $Q_3$ den Hydroxylrest darstellt und $Q_5$ den Caffeoylmaloylrest der Formel (IVa) oder der Formel (IVb) darstellt;
  und

c) - 20,0 Masse-% bis 35,0 Masse-% Wasser,

um das Auftreten von unästhetischen Anzeichen im Zusammenhang mit Entzündungen der Haut und/oder der Kopfhaut beim Menschen zu verhindern oder zu verlangsamen, oder um sie zu beseitigen.

**2.** Zusammensetzung nach Anspruch 1, wobei die Zusammensetzung ($C_1$) für 100% ihrer Masse umfasst:

- von 60,0 Masse-% bis 75,0 Masse-% 1,2-Propandiol,
- von 0,1 Masse-% bis 2,0 Masse-% einer Zusammensetzung (ES), umfassend eine Massenmenge $x_1$, ausgedrückt als Massenäquivalent von 1-O-(2-Caffeoyl)maloyl-3,5-O-dicaffeoylchinasäure, größer oder gleich 200 mg/ von mindestens der Verbindung der Formel (Ia), und von mindestens der Verbindung der Formel (Ib), und von mindestens der Verbindung der Formel (Ic),
- von 20,0 Masse-% bis 35,0 Masse% Wasser.

**3.** Zusammensetzung ($C_2$) umfassend mindestens einen kosmetisch verträglichen Hilfsstoff (E) und eine Zusammensetzung ($C_1$) nach Anspruch 2.

**4.** Zusammensetzung nach Anspruch 3, **dadurch gekennzeichnet, dass** sie zur topischen Anwendung bestimmt ist.

**5.** Zusammensetzung ($C_1$) wie in Anspruch 1 definiert, zu ihrer Verwendung in einem therapeutischen Behandlungsverfahren, das darauf abzielt, Kribbeln und/oder Ameisenlaufen und/oder Juckreiz und/oder Wärmegefühl und/oder Rötungen und/oder Hautunbehagen und/oder Spannungsgefühl der Haut, verursacht durch Entzündung der

menschlichen Haut und/oder Kopfhaut, zu verringern und/oder zu beseitigen.

6. Zusammensetzung ($C_1$) nach Anspruch 5, **dadurch gekennzeichnet, dass** das Kribbeln und/oder Ameisenlaufen und/oder Juckreiz und/oder Wärmegefühl und/oder Rötungen und/oder Hautunbehagen und/oder Spannungsgefühl Hautpathologien wie Urtikaria, ekzematöse Dermatitis, Rosazea, Psoriasis, Herpes, Photodermatosen, atopische Dermatitis, Kontaktdermatitis, Lichen, Prurigo, juckende Krankheiten, Fibrosen, Störungen der Kollagenreifung, Sklerodermie, Ekzem begleiten.

## Claims

1. A composition (C1) comprising for 100% of its mass:

a) - From 60.0% by mass to 75.0% by mass of an organic solvent ($SO_1$) selected from 1,2-propanediol, 1,3-propanediol, 1,4-butanediol, 1,3-butanediol, 1,2-butanediol, 2-methyl-2,4-pentanediol, 1,6-hexanediol, 1,8-octanediol, or a mixture of these compounds;
b) - From 0.1% by mass to 2.0% by mass of a composition (ES) comprising a mass quantity $x_1$, expressed as mass equivalent of 1-O-(2-caffeoyl) maloyl-3,5-O-dicaffeoyl quinic acid, greater than or equal to 200 mg/g of at least one compound of general formula (I):

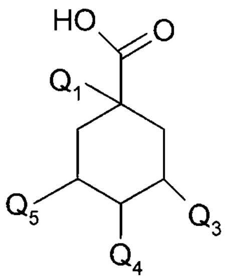
(I),

wherein $Q_1$, $Q_2$, $Q_3$, $Q_4$ and $Q_5$ independently of one another represent the hydroxyl radical or one of its salts or a radical selected from:

(i) - The caffeoyl radical of formula (II) :

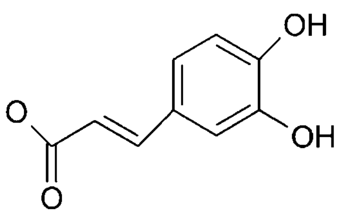
(II)

(ii) - The maloyl radical of formula (IIIa) or (IIIb):

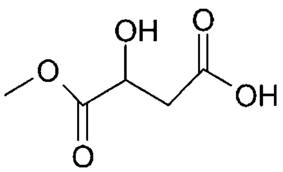
(IIIa)
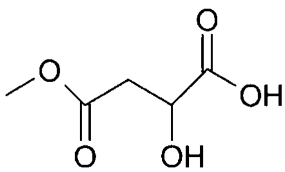
(IIIb) ;

(iii) - The caffeoyl maloyl radical of formula (IVa) or (IVb):

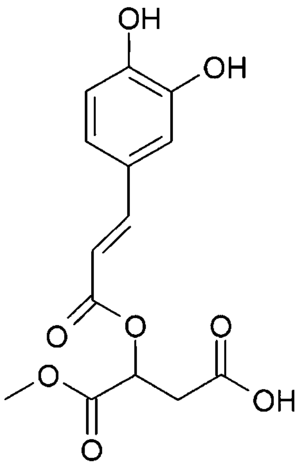
(IVa)

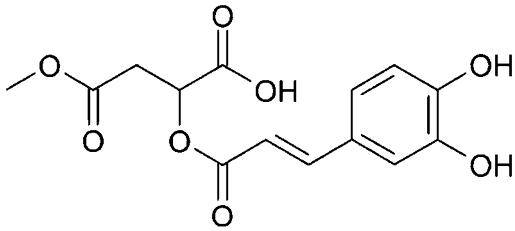
(IVb)

(iv) - The maloyl caffeoyl radical of formula (Va), (Vb), (Vc) or (Vd),

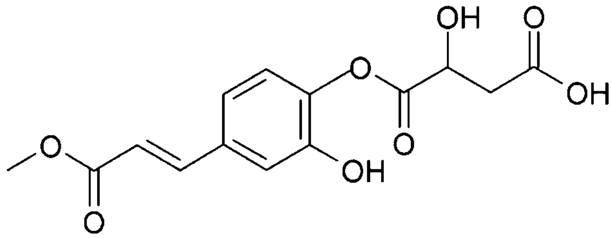
(Va)

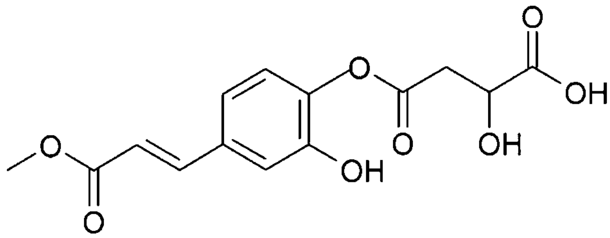
(Vb)

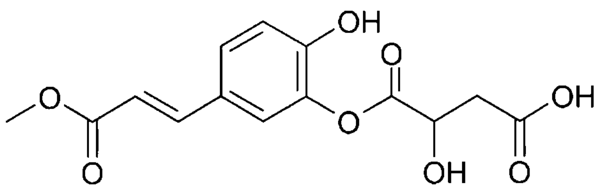
(Vc),

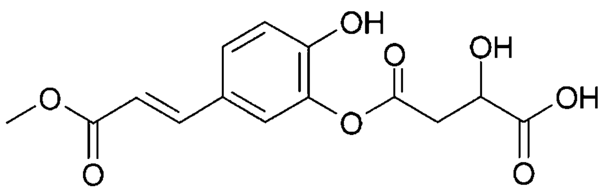
(Vd)

it being understood that at least one of these radicals $Q_1$, $Q_2$, $Q_3$, $Q_4$ and $Q_5$ represents neither the -OH radical; nor one of its salts;
said composition (ES) comprising at least:

- At least one compound of formula (Ia) corresponding to formula (I) for which $Q_1$ represents the maloyl

radical of formula (IIIa) or of formula (IIIb) and $Q_3$ and $Q_4$ and $Q_5$, which are identical, each represent the caffeoyl radical of formula (II);
- A compound of formula (Ib) corresponding to formula (I) for which $Q_1$ represents the caffeoylmaloyl radical of formula (IVa) or of formula (IVb), $Q_3$ and $Q_5$ each represent the caffeoyl radical of formula (II) and $Q_4$ represents the hydroxyl radical, and
- At least one compound of formula (Ic) selected from:

- The compound of formula ($Ic_1$) corresponding to formula (I) for which $Q_1$ and $Q_5$ each represent the caffeoyl radical of formula (II), $Q_3$ represents the hydroxyl radical and $Q_4$ represents the caffeoylmaloyl radical of formula (IVa) or of formula (IVb); and

- The compound of formula ($Ic_2$) corresponding to formula (I) for which $Q_1$ and $Q_4$ each represent the caffeoyl radical of formula (II), $Q_3$ represents the hydroxyl radical and $Q_5$ represents the caffeoylmaloyl radical of formula (IVa) or of formula (IVb);
and

c) - From 20.0% by mass to 35.0% by mass of water,

for preventing or slowing the appearance of unaesthetic signs related to inflammation of the skin and/or scalp in humans, or else for eliminating them.

**2.** The composition according to claim 1, wherein the composition ($C_1$) comprises for 100% of its mass:

- from 60.0% by mass to 75.0% by mass of 1,2-propanediol,
- from 0.1% by mass to 2.0% by mass of a composition (ES) comprising a mass quantity $x_1$, expressed as mass equivalent of 1-O-(2-caffeoyl)maloyl-3,5-O-dicaffeoyl quinic acid, greater than or equal to 200 mg/ of at least the compound of formula (Ia), and of at least the compound of formula (Ib), and of at least the compound of formula (Ic),
- from 20.0% by mass to 35.0% by mass of water.

**3.** A composition ($C_2$) comprising at least one cosmetically acceptable excipient (E) and a composition ($C_1$) according to claim 2.

**4.** The composition according to claim 3, **characterized in that** it is for topical use.

**5.** A composition ($C_1$) as defined in claim 1 for its use in a therapeutic treatment method aimed at reducing and/or eliminating tingling and/or pins and needles and/or itching and/or warming sensations and/or redness and/or cutaneous discomfort and/or tightness of the skin caused by inflammation of human skin and/or scalp.

**6.** The composition ($C_1$) according to claim 5, **characterized in that** the tingling and/or pins and needles and/or itching and/or warming sensations and/or redness and/or cutaneous discomfort and/or tightness accompany cutaneous pathologies such as urticaria, eczematous dermatitis, rosacea, psoriasis, herpes, photodermatoses, atopic dermatitis, contact dermatitis, lichen, prurigos, pruritic diseases, fibroses, disorders of collagen maturation, scleroderma, eczema.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006127525 A2
- CN 106074663 A
- US 20170136077 A
- WO 9600719 A
- EP 0971683 A
- EP 1515688 A2
- WO 9809611 A
- WO 9426694 A
- EP 0977626 A
- WO 2005040230 A


### Littérature non-brevet citée dans la description


- **CHAN et al.** A review of the pharmacological effects of Arctium lappa. *Inflammopharmacol*, 2011, vol. 19, 245-254
- **PIRVU et al.** Comparative studies on analytical, antioxidant, and antimicrobial activities of a series of vegetal extracts prepared from eight plant species growing in Romania. *J planar Chromato*, 2014